# EUROPEAN PATENT SPECIFICATION

(11) **EP 3 191 167 B1**
(45) Date of publication and mention of the grant of the patent: **03.07.2019**
(21) Application number: 14765915.5
(22) Date of filing: 10.09.2014
(51) Int. Cl.: A61M 39/22, F16K 11/083, F16K 11/085

(54) **VALVE FOR ADMINISTRATION OF DRUG FLUIDS**
VENTIL ZUR VERABREICHUNG EINES FLUIDEN MEDIKAMENTS
VALVE POUR L'ADMINISTRATION D'UN MEDICAMENT FLUIDE

(43) Date of publication of application: 19.07.2017
(73) Proprietor: CYTO365 AB, 252 67 Helsingborg (SE)
(72) Inventor: TÖRNBLOM, Micael, 263 65 Viken (SE)
(74) Representative: AWA Sweden AB
(86) International application number: PCT/EP2014/069275
(87) International publication number: WO 2016/037646

(56) References cited:
- WO-A1-2013/146752
- WO-A2-2006/025054
- DE-A1- 3 503 044

## Description

### FIELD OF THE INVENTION

The present inventive concept generally relates to administration of drug fluids. More specifically, the present inventive concept relates to valves or stopcocks for administration of one or more drug fluids, such as cytostatics.

According to one aspect, the inventive concept may be implemented in valves for administration of multiple drug fluids. In the following, such valves will be referred to as multiple-drug valves. In other aspects, the inventive concept may be implemented in conventional stopcocks having two inlets and one outlet.

US 7 984 730 discloses a stopcock comprising a housing and a valve member having in different valve positions. In one position, the stop cock allows flushing of an internal volume of a drug port. The flushing is accomplished by a fluid flow guide arrangement. More specifically, in the flushing operation, the fluid passes from an inlet port into a first end of a first channel formed in the rotary valve member. In the flushing position, this first channel extends from the inlet port towards the port to be flushed. A flow guide integrally formed with the valve member is arranged at the other end of the first channel. In the flushing position, the fluid flow guide is so positioned at the port to be flushed that the port opening is bifurcated by the flow guide. The fluid in the first channel is guided by the flow guide into the bifurcated port. Since the port is bifurcated by the flow guide, the fluid may, after having flushed the port, exit into a second channel in the valve member towards an outlet port.

When the stopcock disclosed in US 7 984 730 is to be used for administering a drug, the rotary valve member is rotated 90 degrees into a different valve position where the inlet of the first channel is now instead aligned with the flushed port and the flow guide is in a different position where it now bifurcates the outlet port instead. In this drug position, a drug may be administered from the flushed port via the first channel to the outlet port. As an alternative, it is suggested in US 7 984 730 to use the flushing position also as a drug administration position in combination with a pressurized fluid at the other inlet. In such an alternative drug administration position, the inlet port for the drug fluid is thus bifurcated.

A problem with this known valve is that the flushing possibility provided results in a compromise with respect to the flow path in other positions. The flow path for administration of drugs is substantially restricted by the ports being bifurcated. Especially, in the 90 degree shifted drug position, the flow guide creates a substantial, unwanted flow restriction for the drug flow. It is generally desirable to provide an essentially unrestricted drug flow, e.g. a drug flow through a flow path having an essentially constant cross section.

Also, in positions where a neutral solution only is to be administered by the valve disclosed in US 7 984 730, the fluid flow guide between the first and the second channel acts as an even more substantial barrier for a free flow, and again compromise design solutions are proposed, resulting in a very restricted flow of neutral fluid.

With respect to prior-art multi-drug valves, there are plenty of medical situations in which a multiple of drug fluids to be inserted into a patient has to be handled. Typically, the various drug fluids are selected and branched by means of a drug-fluid valve. Moreover, in chemotherapy it is often of utmost importance to handle drug fluids to a patient which is treated for cancer in a reliable and safe manner. However, due to stressful working environments, tiredness, the human factor, etc., the handling of these drug fluids, including their connections, dosages, etc. may lead to errors. For example, there is a need to clearly separate different fluids from each other, since they may chemically react in an undesired manner.

Applicant's WO 2013/055278 discloses a multiple-drug valve for administration of a plurality of drug fluids, such as cytostatics. This known multiple-drug valve comprises a housing having a plurality of circumferentially distributed primary inlets for receiving a respective one of the drug fluids and a secondary inlet for receiving a secondary fluid, such as a neutral fluid. The valve has an outlet from which the fluids will be directed to the patient. A rotary valve member is arranged in the housing. The housing has a plurality of primary valve positions in each of which an associated one of the primary inlets is connected to the outlet, and a plurality of intermediary valve positions in each of which the secondary inlet is connected to the outlet. Moreover, the valve member has a outer surface sealingly engaging an inner surface of the housing, such that the primary and secondary inlets are sealingly connected to openings arranged in the outer surface of the valve member in each of the primary and intermediary valve positions, respectively. Further prior art vales are disclosed in EP 2 689 797 A1, WO 2006/025054 A2 and DE 35 03 044 A1.

### SUMMARY OF INVENTION

It is an object of the invention to provide an enhanced valve for administering one or more drug fluids.

According to a first aspect of the inventive concept, there is provided:
a valve for administration of drug fluids, such as cytostatics, comprising:
a valve housing having at least one primary inlet, termed "drug inlet" for receiving a drug fluid and a secondary inlet, termed "saline inlet" for receiving a secondary fluid, such as a neutral fluid;
an outlet; and
a valve member arranged to be positioned, e.g. rotated, into different valve positions in the housing and having a primary channel and a secondary channel,
wherein said different valve positions comprises:
   - at least one primary valve position, termed "drug position", in which the drug inlet is connected to the outlet but not to the saline inlet, and
   - at least one priming/flushing position, in which the saline inlet is connected to the outlet via the drug inlet for priming or flushing the valve with said neutral fluid, and
wherein:
   in the drug position, the drug inlet is connected to the outlet via the primary channel and there is no connection between the drug inlet and secondary channel, and
   in the priming/flushing position, the saline inlet is connected to the drug inlet via the secondary channel and the drug inlet is connected to the outlet via the primary channel.

The inventive concept offers several advantages over the prior art: Since the drug (or drugs in the multiple-drug embodiments) is administered in one channel only - the primary channel or drug channel - and the drug is prevented from entering into the secondary channel, at least the following effects are obtained:
1. According to the inventive concept, the secondary channel is blocked and inactive in the drug position. The primary and secondary channels are so arranged that there is no connection between the drug inlet and secondary channel in the drug position. Since the drug fluid (or fluids) will then never enter the secondary channel in the drug position, it may be possible to manufacture the drug channel differently from the saline channel. For instance, the inside of the drug channel may be manufactured from a different material being more resistant to the drugs being used.
2. Even if a highly resistant material should be used for the whole valve, the inventive concept will offer the advantage that the drug exposed surface is substantially reduced.
3. Reduced risk of drug residuals in the valve member.
4. Using the same channel - the primary channel - between the drug inlet and the outlet both in the drug position and the priming/flushing position has the advantage that the drug channel may be aligned to a higher degree with both the drug inlet and the outlet compared to the prior art. In the prior art, there is a compromise needed, which is not the case in the present invention. An unrestricted drug flow path may be achieved according to the invention, or at least a substantially less restricted flow path, in the drug position compared to the flow path in the priming/flushing position.

In one embodiment, the primary channel is aligned with the drug inlet and with the outlet to a higher degree in the drug position than in the priming/flushing position, whereby a less restricted flow path is established from the drug inlet to the outlet in the drug position than in the priming/flushing position.

In one embodiment, said different valve positions further comprises:
- at least one secondary valve position, termed "saline position", which differs from the drug position and the priming/flushing position and in which the saline inlet is connected to the outlet and there is no connection between the drug inlet and the outlet.

In one embodiment, in the saline position, the saline inlet is connected to the outlet via the primary channel and not connected to the secondary channel. In an alternative embodiment, in the saline position, the saline inlet is connected to the outlet instead via the secondary channel and not connected to the primary channel.

In a preferred embodiment, the priming/flushing position is accomplished by a wall portion or wall portions,
wherein the valve member further comprises a first wall portion arranged between the primary channel and the secondary channel, and
wherein:
- in the priming/flushing position, said first wall portion bifurcates the drug inlet,
- in the drug position, said first wall portion is in sealingly engagement with the housing to prevent any fluid communication between the primary channel and the secondary channel past the first wall portion.

In a preferred embodiment, which makes it possible to achieve a greater positional shift between the drug position and the priming/flushing position, use is made of an additional secondary channel part in the valve member and a by-pass opening in the housing,
wherein the valve member further comprises:
- a fluid transfer channel arranged between the secondary channel and the primary channel,
- a first wall portion being arranged between the fluid transfer channel and the secondary channel, and
- a second wall portion being arranged between the fluid transfer channel and the primary channel,
wherein the housing further comprises an opening, termed by-pass opening, which is formed in an inner surface of the housing and opens towards the valve member, and
wherein
- in the drug position, at least the second wall portion is sealingly engaging the housing to prevent any fluid communication between the primary channel and the secondary channel, and
- in the priming/flushing position, said first wall portion bifurcates the drug inlet, said the second wall portion bifurcates the by-pass opening and the primary channel is connected to the by-pass opening, such that the neutral fluid in the priming/flushing position may flow in the following sequence: the secondary channel → the drug inlet past the first wall portion → the fluid transfer channel → the by-pass opening past the second wall portion → the primary channel → the outlet.

Thus, in this embodiment, which may be implemented both in valves having one primary inlet only and valves having multiple primary inlets, the positional shift from the drug position to the priming/flushing position may be substantially greater compared to the embodiment including only one wall portion. In the embodiment including only one wall portion, the positional shift is restricted by the dimensions of the opening at the drug inlet being primed/flushed. This embodiment has the advantage that it is easier for the user to identify the different positions. For example, if a valve with a rotary valve member presents four drug positions and four priming/flushing positions, interlaced with the drug positions, the angular shift between each position could be around 45 degrees.

The inventive concept may be implemented also in multi-drug valves of the kind disclosed in applicant's WO 2013/055278. In such an embodiment there is provided a valve as defined above,
wherein said at least one primary inlet comprises a plurality of circumferentially distributed primary inlets, termed "drug inlets" for receiving a respective one of said drug fluids,
wherein said at least one primary inlet comprises a plurality of primary inlets, termed "drug inlets" for receiving a respective one of said drug fluids,
wherein said at least one primary valve position comprises:
- a plurality of primary valve positions, termed "drug positions" in each of which an associated one of the drug inlets is connected to the outlet via the drug channel, and
wherein said at least one priming/flushing position comprises:
- a plurality of priming/flushing positions, in which the saline inlet is connected to the outlet via an associated one of the drug inlets.

In a multi-drug embodiment of the inventive concept, the priming/flushing positions may be used for administration of the neutral fluid also. However, in a preferred embodiment, said different valve positions may also comprise a plurality of secondary positions or "saline positions", which differ from said plurality of drug positions and said plurality of priming/flushing positions and in which the saline inlet is connected to the outlet. The different valve positions are preferably so arranged that there is one priming/flushing position and one saline position between two drug positions. For instance, if the valve member is a rotatable valve member and is rotated in a given direction, the position sequence may be as follows:
Priming → Drug position #1 → Flushing position #1 → Saline position #1 → Drug position #2 → Flushing position #2 → Saline position #2 → etc.

As an example, in a rotary multi-drug valve having four drug positions circumferentially distributed at 90 degree intervals, the four saline positions may also be circumferentially distributed at 90 degree intervals, but shifted 45 degrees in relation to the drug positions. Using the technique disclosed above involving the transfer channel and the by-pass opening, the four priming/flushing positions could also be circumferentially distributed at 90 degree intervals but shifted at an angle of e.g. 15 degrees in relation to the drug positions.

A valve according to the inventive concept may be designed as a single-level valve or a multi-level valve. In the single-level configuration, the inlets and the outlet are arranged in common plane, typically perpendicular to a rotational axis of the valve member. In the multi-level configuration, the inlets and the outlet are arranged in at least two different levels. Multi-drug configurations would typically be also multi-level configurations.

The outlet from the valve may be in the same plane or level as the inlets, but it may also be directed towards the bottom of the valve and centered in relation to a rotational axis of the valve member. The outlet may be formed in the housing or directly in the valve member.

The different valve positions are preferably predetermined positions and are preferably identifiable by the user. This may be done by the shape of a handle member as known, but also by arranging a tactile response to a user at each valve position ("click indication").

According to an example, there is provided a method of operating a valve for administration of drug fluids, such as cytostatics, said valve comprising:
a valve housing having at least one primary inlet, termed "drug inlet" for receiving a drug fluid and a secondary inlet, termed "saline inlet" for receiving a secondary fluid, such as a neutral fluid;
an outlet; and
a valve member having a primary channel and a secondary channel, wherein the valve member when using the valve is positioned in different valve positions, which include at least:
   - a primary valve position, termed "drug position", in which the drug inlet is connected to the outlet but not to the saline inlet, and
   - a priming position, in which the saline inlet is connected to the outlet via the drug inlet
wherein
the primary channel is used for fluid flow both in the drug position and in the priming/flushing position for connecting the drug inlet to the outlet, and
the secondary channel is not used in the drug position.

In some embodiments, the housing is cylindrical, having a cylindrical inner surface, and the valve member is cylindrical, having a cylindrical outer surface.

In preferred embodiments, the valve member is rotatably arranged in said housing.

In preferred embodiments, the primary inlets and/or the secondary inlet and/or the outlet are integrally formed with said housing. Thereby, these parts may be molded simultaneously with the housing, e.g. by injection molding.

The valve is preferably intended for one complete treatment use. The housing and the valve member may be manufactured by means of molding, such as injection molding. More specifically, the housing and the valve member may each be manufactured in a single piece.

### Terminology

The term "drug fluid" as used herein is to be interpreted in a wide sense and should not be limited to pure drugs. Drug fluids may include various types of cytostatics which are to be infused into the vascular system of a patient intravenously in order to treat her/him from cancer. Other fluids which may be administered by the present inventive valve include volume expanders, blood-based products, blood substitutes, medications, nutritional solutions, antibiotics etc.

The term "secondary fluid" and "saline fluid" are used herein as equivalents and are to be interpreted as any suitable fluid to be administered to the patient and/or for priming/flushing purposes. Especially, the secondary fluid may be a neutral fluid, such as a saline solution.

The terms "primary inlet" and "drug inlet" are used herein as equivalents. The terms "secondary inlet" and "saline inlet" are used herein as equivalents".

The terms "primary channel" and "secondary channel" should be construed as including channels in the form of a bore having defined end openings, open recess configurations, or combinations thereof.

The term "connected" with respect to the inlets, the outlet, the channels, etc. is to be construed as "fluidly connected".

The terms ""primary valve position" and "drug position" are used herein as equivalents and are to be construed as an intended position of the valve member different from the prime/flush position in which a drug fluid may be administered from a primary inlet to the outlet.

The terms "secondary position" or "saline position" are used herein as equivalents and are to be interpreted as an intended position of the valve member, different from the drug position in which a neutral fluid may be administered from the saline inlet to the outlet.

The term "priming" as used herein is to be construed as an operation performed for removing air from the valve and providing a fluid-filled valve.

The term "priming/flushing position" is to be construed as a valve position allowing priming or flushing to be performed.

The term "flushing" as used herein is to be construed as the operation performed for cleaning the valve or parts thereof from previously administered drugs. Flushing the valve housing (or parts thereof) would be equivalent to priming the valve housing (or parts thereof). However, the valve member may be flushed without the whole valve being primed.

Other features and advantages of embodiments of the present invention will become apparent to those skilled in the art upon review of the following drawings, the detailed description, and the appended claims.

### BRIEF DESCRIPTION OF THE DRAWINGS

The inventive concept, some non-limiting embodiments and further advantages of the inventive concept will now be further described with reference to the drawings.

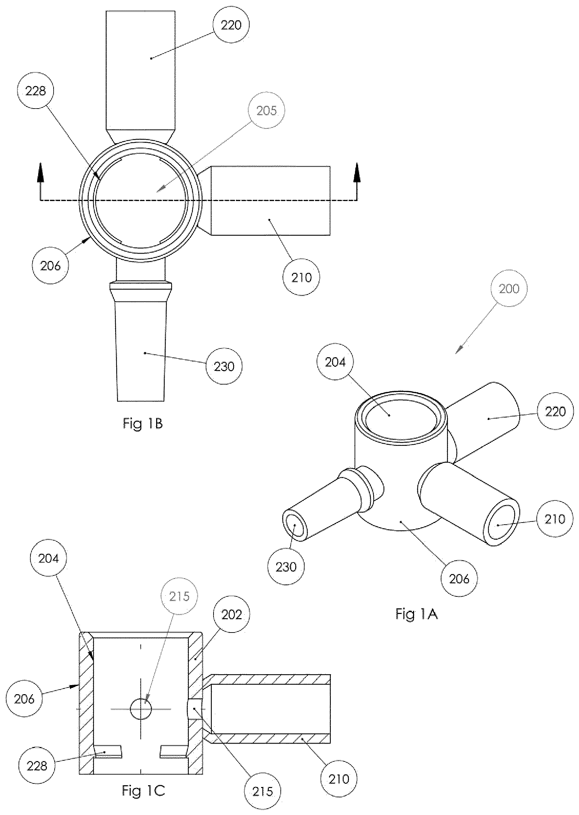

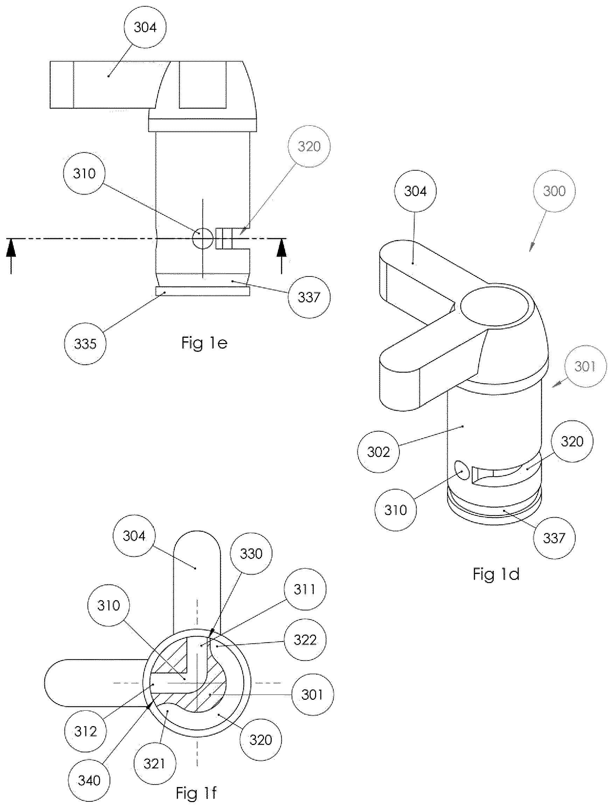

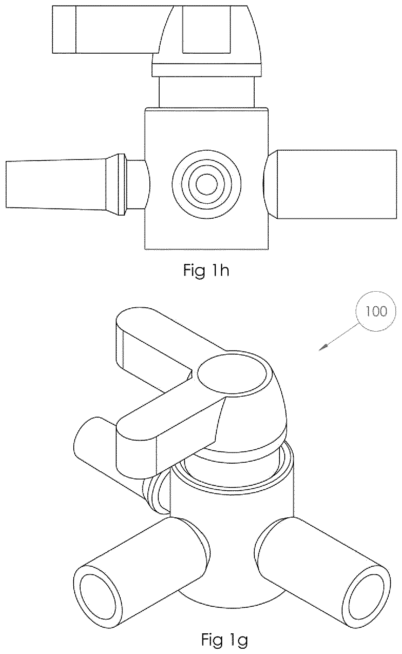

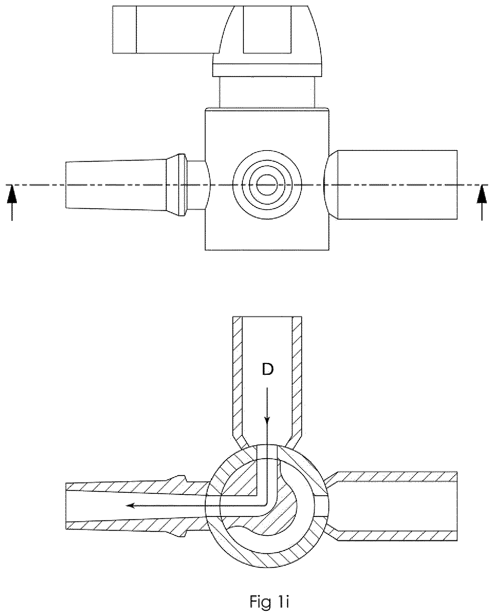

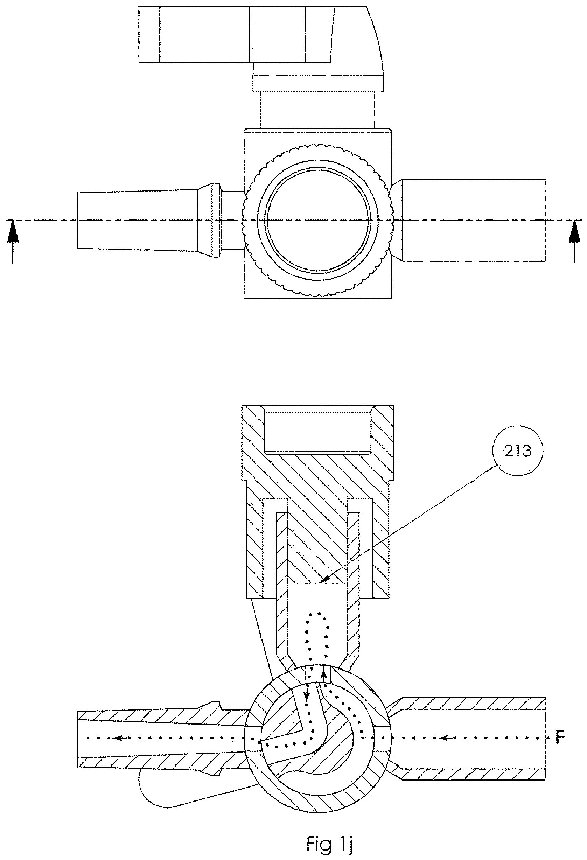

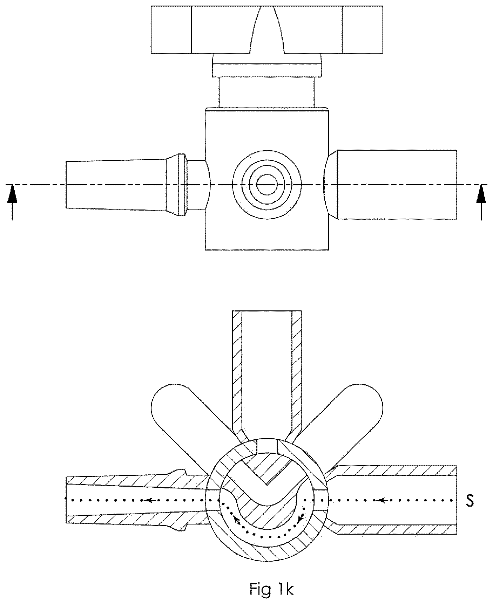

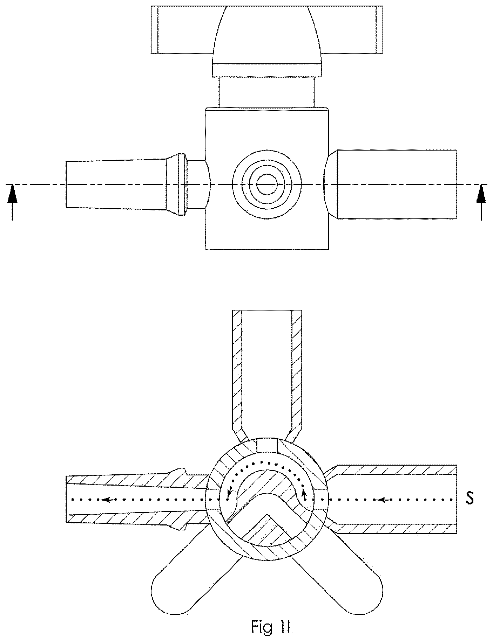

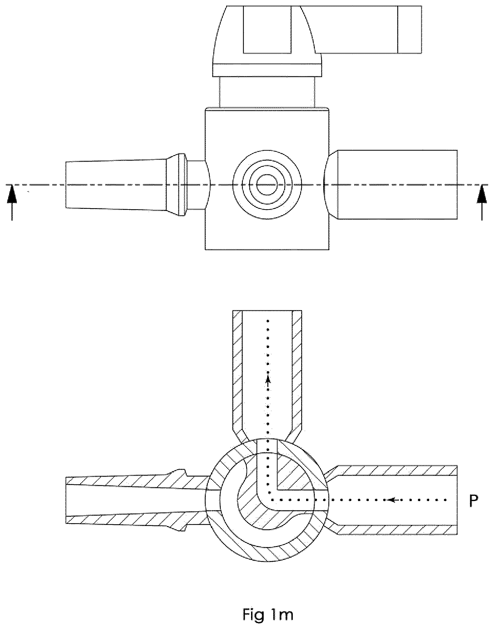

Each embodiment is illustrated with a number of figures marked with letters (e.g. Fig. 1a, Fig. 1b, etc for the first embodiment). In the following, any reference to the figure number only (e.g. Fig. 1) is a general reference to all the individual figures for the corresponding embodiment.
- Fig. 1: illustrates a first embodiment of a single-level valve.
- Fig. 2: illustrates a second embodiment of a single-level valve.
- Fig. 3: illustrates a first embodiment of a multi-level valve.
- Fig. 4: illustrates a second embodiment of a multi-level valve.
- Fig. 5: illustrates a third embodiment of a multi-level valve
- Fig. 6: illustrates a fourth embodiment of a multi-level valve.
- Fig. 7: illustrates a fifth embodiment of a multi-level valve.
- Fig. 8: illustrates a sixth embodiment of a multi-level valve with an alternative outlet direction.
- Figs 9: illustrates a first embodiment of a multi-level, multi-drug valve.
- Fig. 10: illustrates a second embodiment of a multi-level, multi-drug valve.
- Fig. 11: illustrates a third embodiment of a multi-level, multi-drug valve.
- Fig. 12: illustrates an alternative embodiment of a single-level valve.

The present inventive concept relates to disposable valves for administration of drug fluids. For example, the drug fluids may include various types of cytostatics which are to be infused into the vascular system of a patient intravenously in order to treat her/him from cancer. Other fluids which may be administered by the present inventive valve include volume expanders, blood-based products, blood substitutes, medications, nutritional solutions, etc.

In one example, a drip chamber may be provided before the valve. Preferably, one drip chamber is provided in connection to each bag storing the fluids.

In addition, a booster pump may be utilized in order to ensure that the drip chamber never becomes empty of fluid. The booster pump may maintain a predetermined pressure in the tube, and may thereby prevent gas from passing into the bloodstream of the patient. In one example, the booster pump is provided before the valve. In another example, the booster pump is provided after the valve.

### First embodiment of a single-layer valve

Reference is now made to Fig. 1 which illustrate a first embodiment of a valve or stopcock 100 according to the inventive concept for administration of drug fluids. This embodiment is termed "single-level" since the inlets and outlets are arranged in a common plane or level, as opposed to other embodiments termed "multi-level".

The valve 100 comprises a cylindrical housing 200 (Figs. 1a to 1c) and a valve member 300 (Figs. 1d to 1f). A cylindrical body 301 of the valve member is rotatably arranged in the housing 200. By cylindrical is here meant a cylinder-shape with constant radius. The housing 200 and the valve body 301 may have shapes other than cylindrical. For example, they may be frustoconical, comprised of several frustoconical parts, etc. More specifically, an outer cylindrical surface 302 of the valve body 301 engages sealingly with an inner cylindrical surface 204 of the housing 200, thereby creating an assembly which is airtight and prevents the flow of fluids. Preferably, the diameter of the outer cylindrical surface 302 of the valve member 300 is slightly larger than the inner cylindrical surface 204 of the housing 200 in order to create the sealed engagement mentioned above.

Alternatively, other ways of engaging sealingly is conceivable. In one example, a sealing element (not shown) may be arranged in between the outer cylindrical surface 302 of the valve member 300 and the inner cylindrical surface 204 of the housing 200. For example, the sealing element may be made of a thin, flexible material which may be fitted snugly between the valve member and the housing. For example, the sealing member may be an O-ring.

The housing 200 and the valve member 300 may be fabricated in any material which does not react chemically to any considerably extent with the intended drug fluids to be used, and which thereby and also in other aspects is suitable for medical applications. Moreover, the material must be suitable for sterile environments. Examples of materials include plastic materials. The plastic material may be transparent or opaque depending on the medical application.

The housing 200 may be fabricated in the same material as the valve member 300. Alternatively, the housing 200 may be fabricated in a material which is different from that of the valve member 300. In particular, the material of the housing 200 and the valve member may have different hardness.

The housing 200 illustrated in Figs 1a to 1c. comprises a cylindrical housing wall 202 which encloses an inner cavity 205 into which the valve member 300 is to be inserted. The cylindrical housing wall 202 comprises an outer envelope surface 206 and said inner cylindrical surface 204. The housing 200 comprises a primary inlet 210, a secondary inlet 220 and an outlet 230. All three connects to the inner cavity 205 of the housing 200 and may be integrally formed with the cylindrical housing wall 202 and shaped as a pipe or a stud. Since the primary inlet 210 is intended normally to be used for the administration of drugs and the secondary inlet 220 is intended normally to be used for the administration of a neutral fluid, such as a saline solution or saline fluid, these inlets will for explanation purposes be referred to as "the drug inlet 210" and "the saline inlet 220", respectively, without therefore limiting the scope of the claims to the use of any specific fluids.

In the embodiment shown in Fig. 1, the drug inlet 210, the saline inlet 220 and the outlet 230 are circumferentially distributed in a T-configuration in a common geometrical plane In other embodiments, they may have different mutual positions in the T-configuration and/or be arranged in different levels or planes as will be described below. Other configurations than T-configurations are also possible as will be described below.

In use, a neutral fluid, such as a saline solution, or equivalently a saline fluid, may be is led into the saline inlet 220 by means of a tube. The saline solution may comprise a sterile solution of sodium chloride (NaCI). The saline inlet 220 may be provided with a saline connection device (not shown) for connecting said tube. In a non-limiting example, saline connection devices comprises male and female Luer connectors. It is clear, however, that any type of saline connection devices may be used.

The valve member 300 (Figs 1d-1f) is integrally formed with said cylindrical body 301 and a V-shaped handle 304 arranged outside the housing 200 by means of which the valve member 300 may be rotated by the user. The V-shape will in known manner allow the user to identify the position of the valve member 300 and it will be noted that the sectional views illustrating the different valve positions also show the position of the V-shaped handle 304.

The body 301 of the valve member 300 which is rotatably and sealingly arranged in the inner cavity 205 of housing 200 is provided with two fluid channels: a primary channel 310 and a secondary channel 320. As will be discussed below, according to the inventive concept the primary channel 310 is intended for primarily for drugs and the secondary channel 320 is intended primarily for neutral fluids, such as a saline solution or saline fluid. Therefore, the primary channel 310 and the secondary channel 320 of the valve member 300 will for explanation purposes be referred to as "the drug channel 310" and "the saline channel 320", respectively, without therefore limiting the scope of the claims to the use of any specific fluids therein.

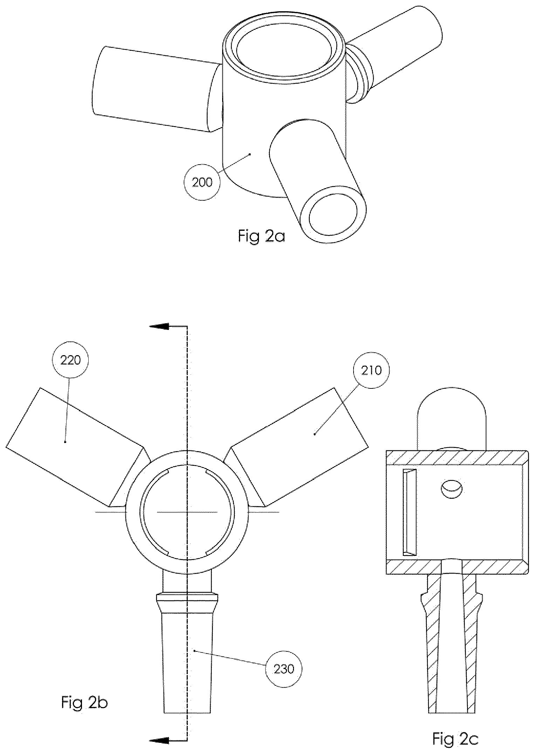

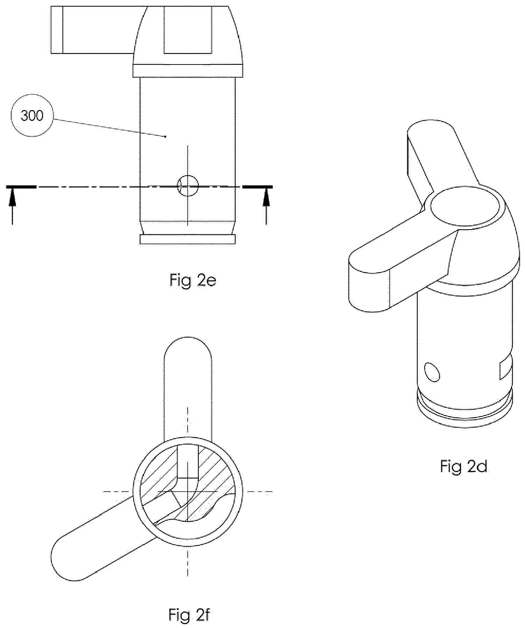

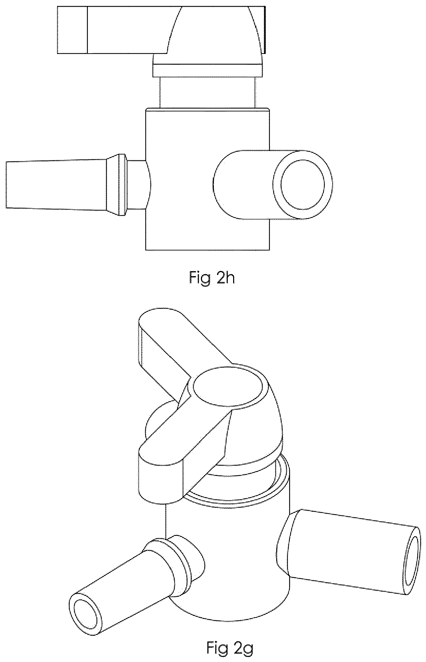

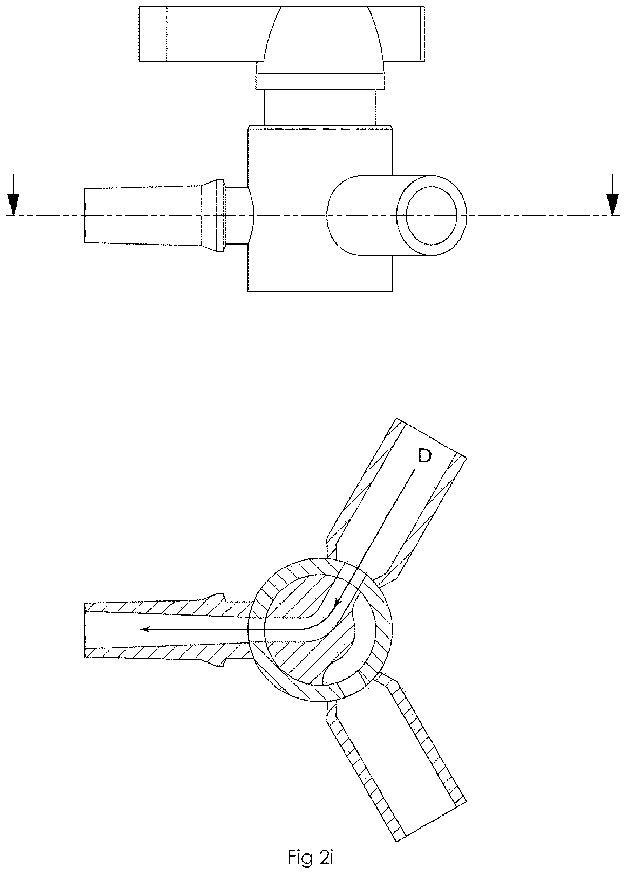

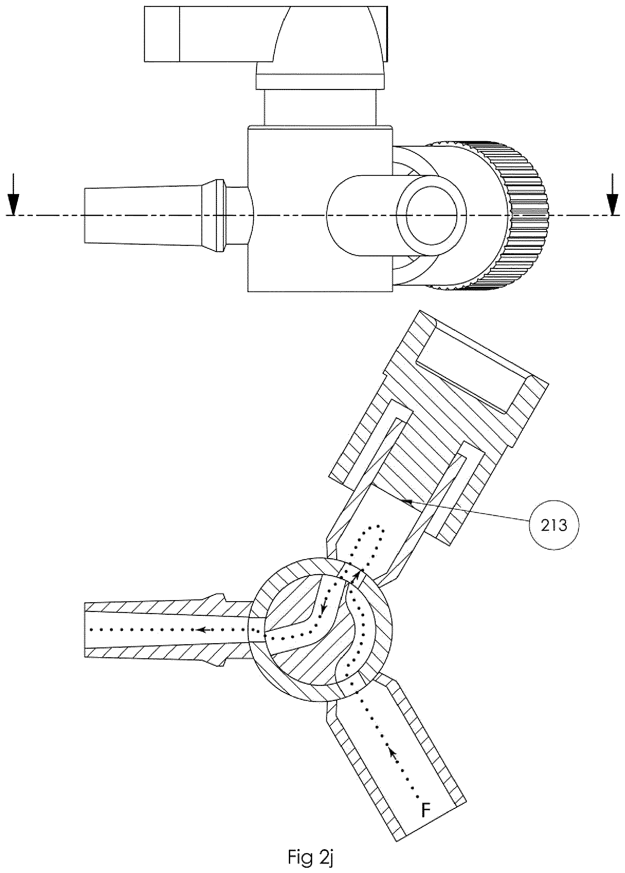

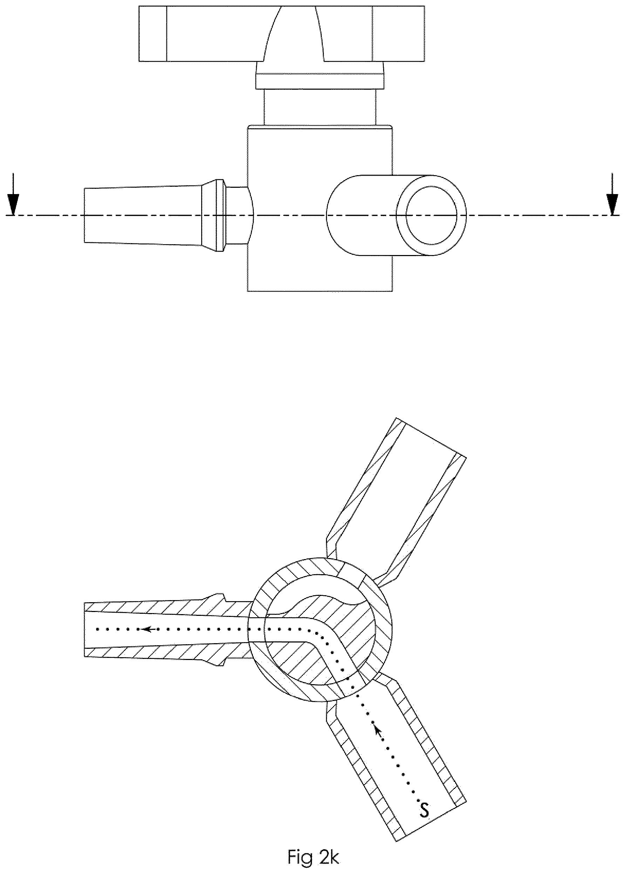

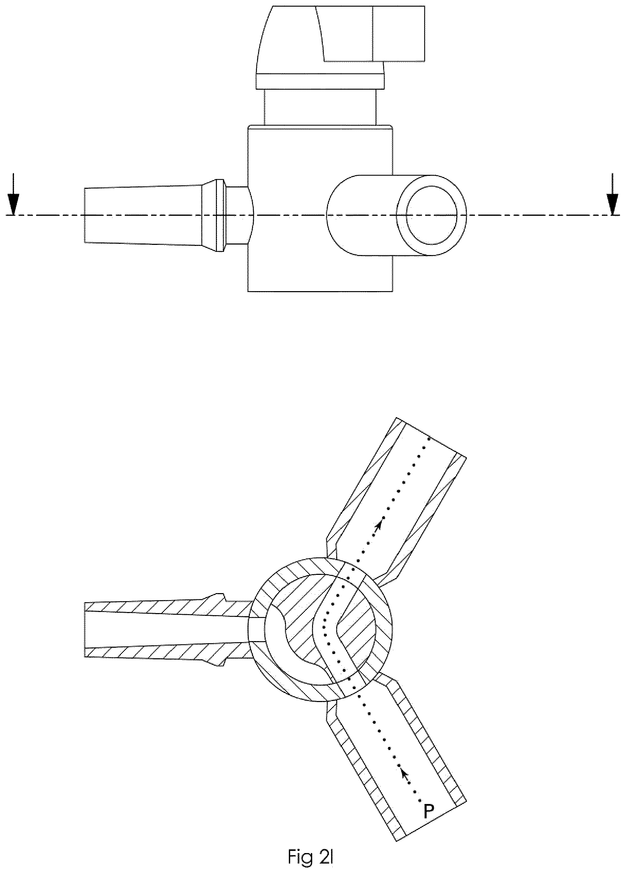

In the embodiment illustrated in Fig. 1, the drug channel 310 is formed as a bore through the body 301 and presents first and second ends or openings 311 and 312, respectively. In the illustrated embodiment, the drug channel 310 is a 90 degree channel configured to be fluidly connected to or aligned with the inlets 210 and 220 and the outlet 230 of the valve in the manner to be described. Accordingly, in other embodiments where the configuration of the inlets and the outlet differs from a T-configuration, the drug channel 310 may be configured accordingly. Such an embodiment will be described later with reference to Fig. 2. The actual geometrical path of the drug channel 310 inside the body 301 may not be critical and may differ from the 90 degree bend configuration shown. The position of the openings 311 and 312 of the drug channel 310 is more important.

The saline channel 320 is formed as a circumferential oriented recess in the body 301 open towards the outer cylindrical surface 302 and presents two ends 321 and 322 which are circumferentially spaced about 180 degrees.

The drug channel 310 and the saline channel 320 do not communicate with each other directly within the valve member 300. As shown in the sectional views in

Fig. 1, the relative angular positions of the drug channel 310 and the saline channel 320 of the valve member 300 is such that the first end 311 of the drug channel 310 and the second end 322 of the saline channel 320 are separated by a relatively narrow (in the circumferential direction) first wall portion or wall portion 330. The other ends of the drug channel 310 and the saline channel 320 are separated by a somewhat broader second wall portion or wall portion 340. The wall portions 330 and 340 are preferably integrally formed with the body 301 of the valve member 300.

Reference numeral 228 indicates a inwardly directed flange or rim on the inner surface of the housing. Reference numeral 337 indicates a corresponding annular recess in the valve body 301 which receives the flange 228 in the assembled state in order to maintain the valve member 300 in the housing 200.

The two wall portions 330 and 340 extend essentially radially, and when the valve member 300 is in a rotary position in which they are directed towards the inner cylindrical surface 204 there is a sealing engagement between the outer edge of the wall portions 330, 340 and the inner cylindrical surface 204.

The operation of the valve in Fig. 1 will now be described with reference to the sectional views in Fig. 1 illustrating the following valve positions:
- Fig. 1i: Drug administration
- Fig. 1j: Priming/Flushing
- Fig. 1k: Saline administration
- Fig. 1l: All open position
- Fig. 1m: Flushing IV set

Fig. 1i illustrates the drug position of the valve 100 in Fig. 1. A source of drug fluid (not shown) will be connected to the drug inlet 210 and the handle 310 has been turned into a position where the openings 211 and 212 of the drug channel 310 are fully aligned with the drug inlet 210 and the outlet 230, thereby creating an unrestricted flow path for the drug fluid as illustrated by a solid arrow D. The term "unrestricted" should be construed as a flow path where the drug channel 310 does not partly block the inflow from the inlet 210 or the outflow to the outlet 220. In the disclosed embodiment, the drug channel 310 has a an essentially constant cross section matching the cross sections of the openings 215 in the cylindrical housing wall 202 at the drug inlet 210 and the drug outlet 230. It should especially be noted that there is no partly blocking of the drug fluid D by the two wall portions 330 and 340, which in the drug position are located at a rotary position away from the inlet and the outlet.

In the drug position (Fig. 1i), the saline channel 320 is inactive. Although it is open towards the saline inlet 220 there would be no flow since the saline channel 320 is blocked at both ends 321 and 322 by the wall portions or wall portions 330 and 340, sealingly engaging the inner cylindrical surface 204 of the housing. Thus, in the drug position the only channel which is active of the two channels 310 and 320 is the drug channel 320.

Turning now to the priming/flushing position illustrated in Fig. 1j, it will be seen that the valve member 300 has been turned a limited angle counter-clockwise compared to the drug position in Fig. 1i. In the priming/flushing position, both the drug channel 310 and the saline channel 320 are in fluid contact with the drug inlet 210. Thus, both channels 310 and 320 are used. The saline channel 320 is also in fluid contact with the saline inlet 220 and the drug channel 310 is also in fluid contact with the outlet 230. More specifically, the radially outer edge of the first wall portion 320 is so aligned with the inlet 210 that it bifurcates the opening 215 in the cylindrical housing wall 202. Obviously, the circumferentially width of the first wall portion 330 will be adapted to the cross sectional area of the opening 215 in order to make the fluid connection possible with both the drug channel 310 and the saline channel 320 at the same time. In this embodiment, the angle of rotation between the drug position and the priming/flushing position is rather limited and will be determined by the cross sectional dimension of the opening 215 in the wall housing 202.

In the priming/flushing position, the drug inlet 210 would typically be blocked, e.g. by a cap or an elastomeric element as schematically illustrated at 213 in Fig. 1j and the saline inlet 220 would typically be connected to a source of a neutral fluid such as a saline fluid. The saline fluid will then follow the flow path indicated by the dashed arrow F, from the inlet 220, through part of the saline channel 320, out in the drug inlet 210, back out in the drug channel 310 and out through the outlet 230. It will be noted that the saline flow path F will be more restricted compared to the drug flow path D since (i) the saline flow F has to follow the bifurcated path at the inlet 210 and (ii) the saline flow S also will encounter a partially blocked situation at the outlet 230 due to the drug channel 310 not being fully aligned with the outlet 230. However, this flow restriction in the priming/flushing position is not crucial, and it is generally more important to achieve a less restricted or preferably a full flow in the drug position.

As indicated by the term "priming/flushing position", this position may be used both for priming and flushing. Priming is normally performed at an initial stage in order to remove air from the valve. Flushing is typically performed after having administered a drug in order (i) to ensure that all drug fluid present in the valve is administered to the patient and (ii) to prevent bacteria growth in the valve.

Turning now to the saline position illustrated in Fig. 1k, the valve member 300 has been turned a into a position where the saline channel 320 creates a direct saline flow path S as indicated by a dashed arrow from the saline inlet 220 to the outlet 230. The wall portions 330 and 340 of the valve member 300 are both in sealingly engagement with the housing wall 202 and there is no flow in the thus inactive drug channel 310. The drug inlet 210 is blocked by the body 301 of the valve member 300. The saline position is typically used after the flushing position. As an example the sequence of operation may be as follows:
Priming → Drug → Flushing → Saline.

It will also be noted that in the embodiment in Fig. 1, the saline channel 320 is so configured that it is not doing any partially blocking at the saline inlet 220 or at the outlet 230. Thus, the saline channel 320 creates an essentially unrestricted saline flow S through the valve in the saline position.

Fig. 1l illustrates another possible valve position, in which the valve member 300 has been rotated 180 degrees compared to the saline position in Fig. 1k. In this "all open position", the drug inlet 210 and the saline inlet 220 are both in fluid connection with the outlet 230 via the saline channel 320, whereas the drug channel 310 is blocked and inactive.

Fig. 1m illustrates yet another possible valve position, in which the valve member 300 has been rotated such that the drug channel 310 creates an unrestricted flow path P between the saline inlet 330 and the drug inlet 310. This valve position may be used for flushing the IV set (not shown) connected to the drug inlet 310.

### Second embodiment of a single-layer valve

Reference is now made to Fig. 2, illustrating a second embodiment of a single-layer valve according to the inventive concept. Same reference numerals are used in Fig. 1 and in Fig. 2 for corresponding parts. The embodiment in Fig. 2 differs from the embodiment in Fig. 1 in that the drug inlet 210, the saline inlet 220 and the outlet 230 are not arranged in a T-configuration but rather equally spaced apart in the circumferentially direction by an angle of about 120 degrees. As an alternative the two inlets may be located closer to each other than 120 degrees. The housing 200 is shown in Figs 2a-c and the valve member 300 is shown in Figs 2d-f.

The operation of the valve in Fig. 2 is essentially the same as for the embodiment in Fig. 1. The positions available are the following (the "all open position" being not available):
- Fig. 2i: Drug administration
- Fig. 1j: Priming/Flushing
- Fig. 1k: Saline administration
- Fig. 1l: Flushing IV set

As will be seen in the sectional views, in this embodiment the drug channel 310 is formed as a 120 degree channel, so that it may be fully aligned at both ends in the drug position (Fig. 2i). It will also be fully aligned in the saline position (Fig. 2k) and the flushing IV set position (Fig. 1l). The saline channel 320 is shorter in the circumferential direction compared to the embodiment in Fig. 1.

### Multi-level valve embodiments

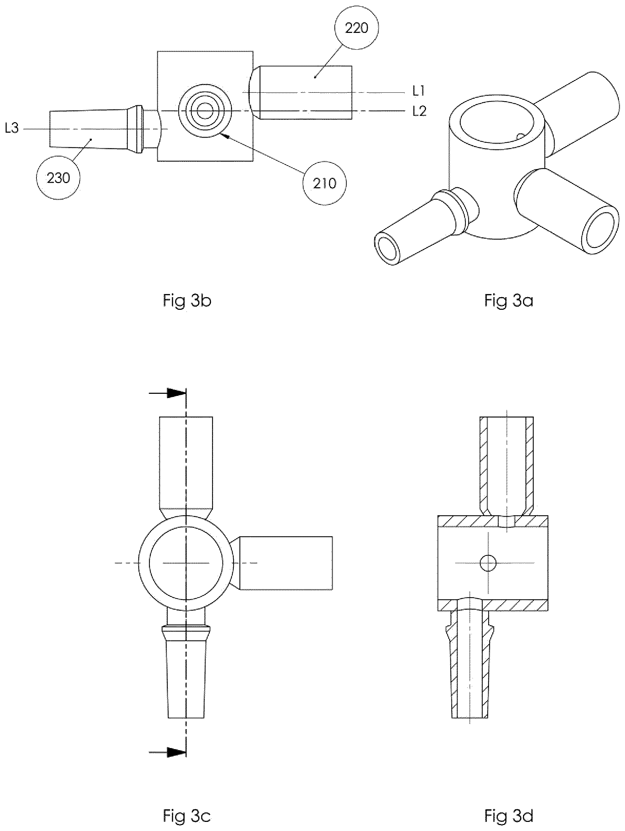

Reference is now made to Figs. 3 to 6 illustrating a number of different embodiments of a multi-level valve according to the inventive concept. These embodiments differ from the embodiments in Figs 1 and 2 in that the drug inlet 310, the saline inlet 320 and the outlet 330 are not located in a common plane or level.

In the embodiment illustrated in Fig. 3, the saline inlet 220 is located on a first level L1, the drug inlet is located on an intermediate level L2 and the outlet 230 is located at a third level L3.

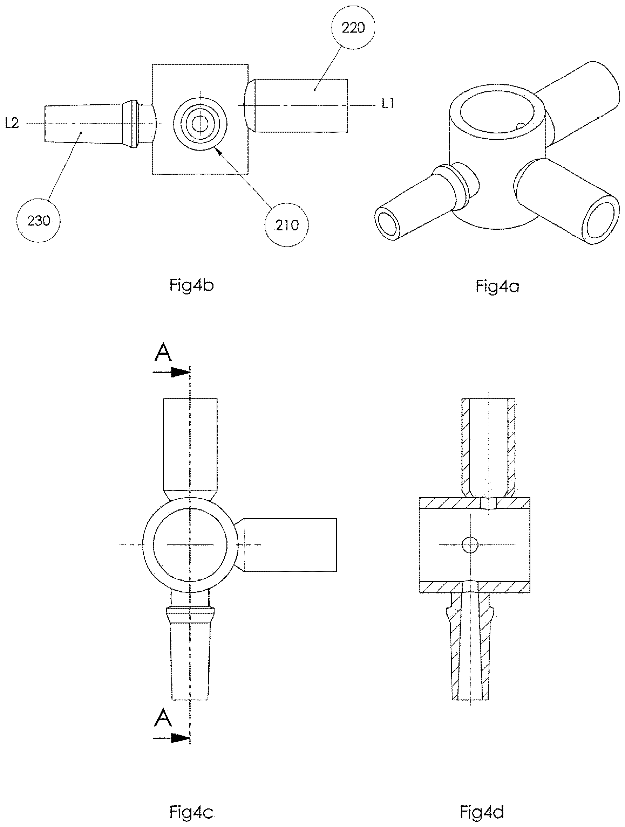

The embodiment in Fig. 4 is a two-level embodiment where the saline inlet 220 is on a first level L1 and both the drug inlet 210 and the outlet 230 are on a second level L2.

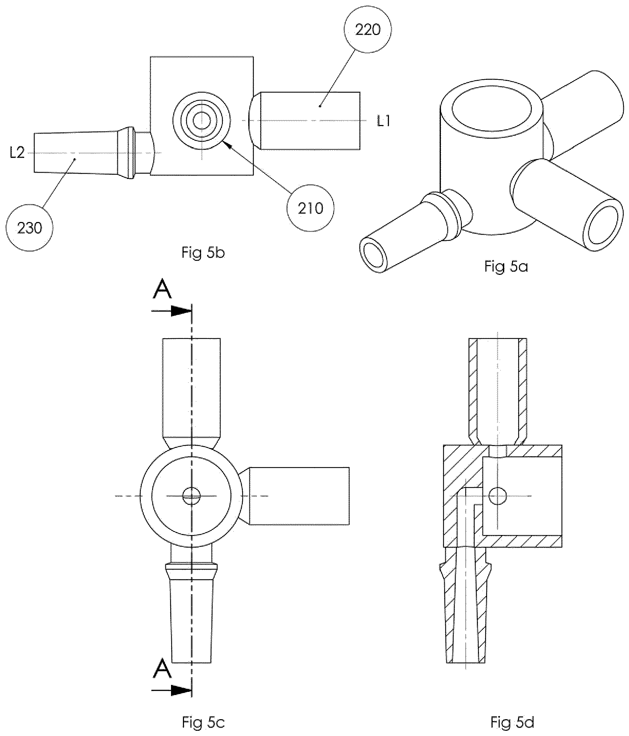

The embodiment in Fig. 5 is also a two-level embodiment, but here the two inlets 210, 220 are on a common level L1 and the outlet 230 is on different level L2.

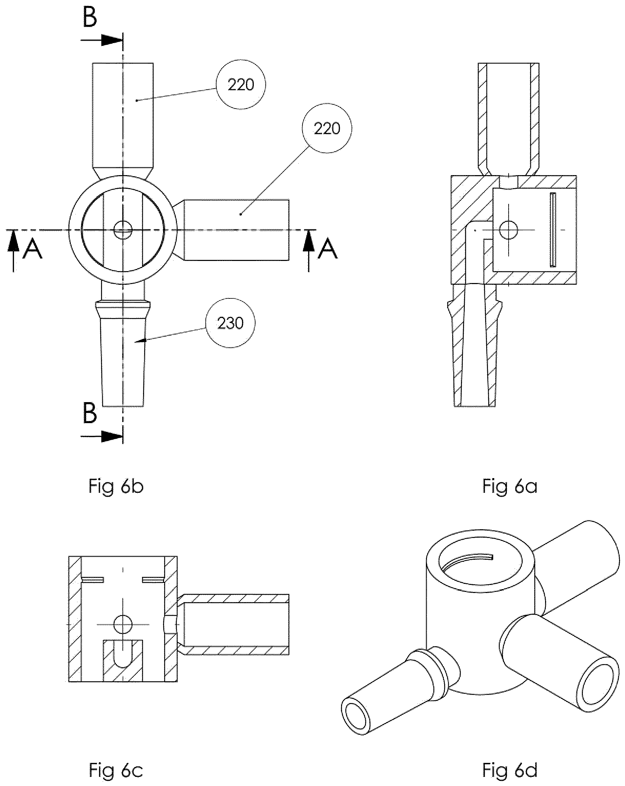

The embodiment in Fig. 6 corresponds to the embodiment in Fig. 5 but the housing has a partially open bottom.

### Embodiment with vertical outlet

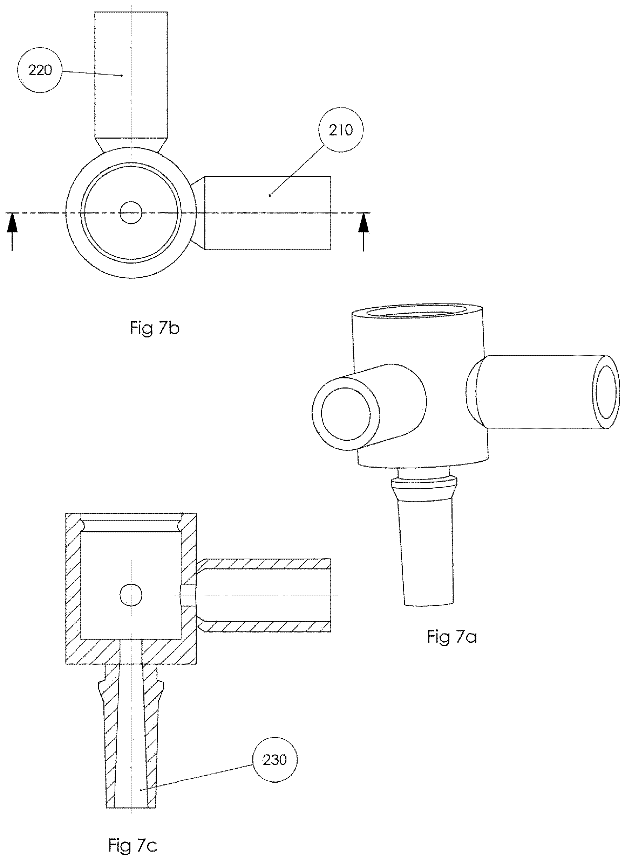

In general, in a valve according to the inventive concept, the outlet 230 does not have to extend in a plane common with or parallel to the inlets. It is possible to arrange the outlet 230 in a different direction. Fig. 7 shows an example where the drug inlet 210 and saline inlet 220 are located in a common plane, perpendicular to the axis of rotation A, and the outlet 230 is centered and extends along the axis A. It is also possible in such a configuration to arrange the outlet directly in the valve member 300 without using a specific port in the housing for the outlet function.

### Simple multilevel valve

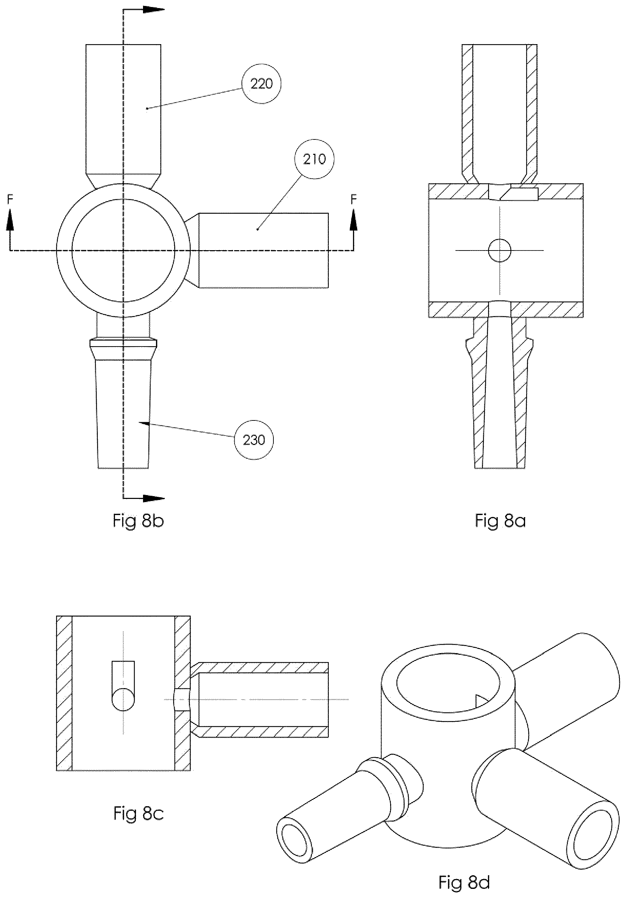

Fig. 8 discloses a simple multilevel valve where the saline fluid entering the saline inlet is guided to another level by means of a recess formed in the housing wall, and then further to an annular recess in the valve member (not shown).

### Multi-drug embodiments

As has been described above and illustrated in connection with embodiments having two inlets and one outlet, the present inventive concept makes it possible to implement a valve which presents both (i) a specific priming/flushing position in addition to two other, rotatably different drug and saline positions, and (ii) the possibility of obtaining an unrestricted drug flow, or at least a less restricted drug flow compared to the priming/flushing flow. This inventive concept may be implemented also in multiple-drug valves, e.g. of the kind as disclosed in applicant's WO 2013/055278 mentioned above. Two such embodiments will be described in the following.

As with the multi-drug valve disclosed in WO 2013/055278 a multi-drug valve according to the inventive concept ensures administration of a neutral fluid in connection with switching from one drug fluid to another, but now also adds the possibility of priming as well as intermittent flushing the valve, and all this while maintaining a full or at least acceptable drug flow in the drug positions.

The drug fluids and the neutral fluid are preferably stored in bags on a rack at levels which are spatially higher than the entry into the vein of the patient, so that that these fluids may pass downwards by means of the gravitational force. Typically, the bags store 500 ml or 1000 ml of fluid. Alternatively, the fluids may be stored in vials or containers of similar types. According to another alternative, the fluids may be passed to the vein of the patient by means of a pump device. Moreover, the fluids are passed in tubes to a valve, which comprises a plurality of fluid inlets for the fluids. The valve will alternately distribute the drug fluids and the neutral fluid, as will be elaborated below. After passing the valve, one of the drug fluids or the neutral fluid is transported to a peripheral intravenous line arranged on the patient by means of a catheter tube. The fluid is thereafter led into the patient by means of a catheter which is placed into a peripheral vein.

### First embodiment of a multi-drug valve

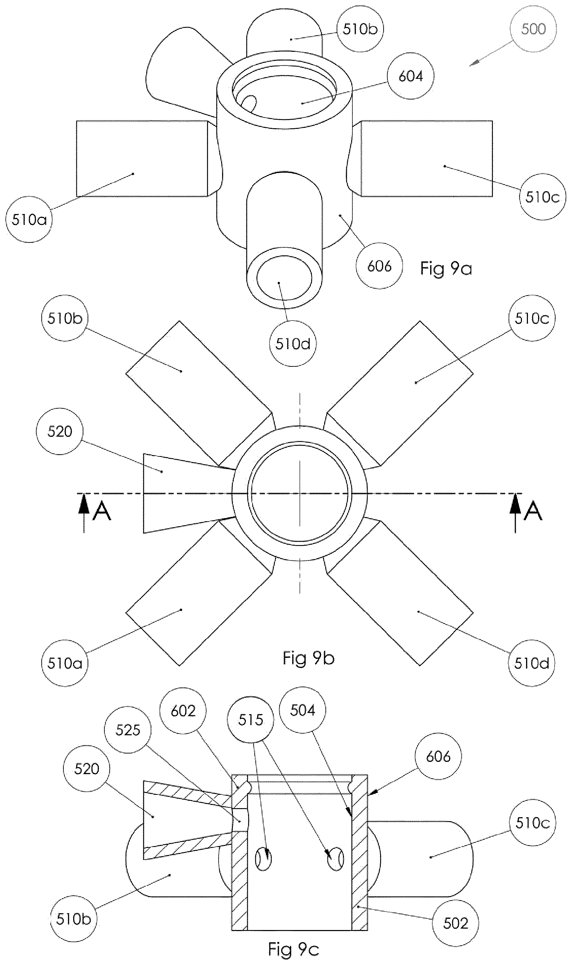

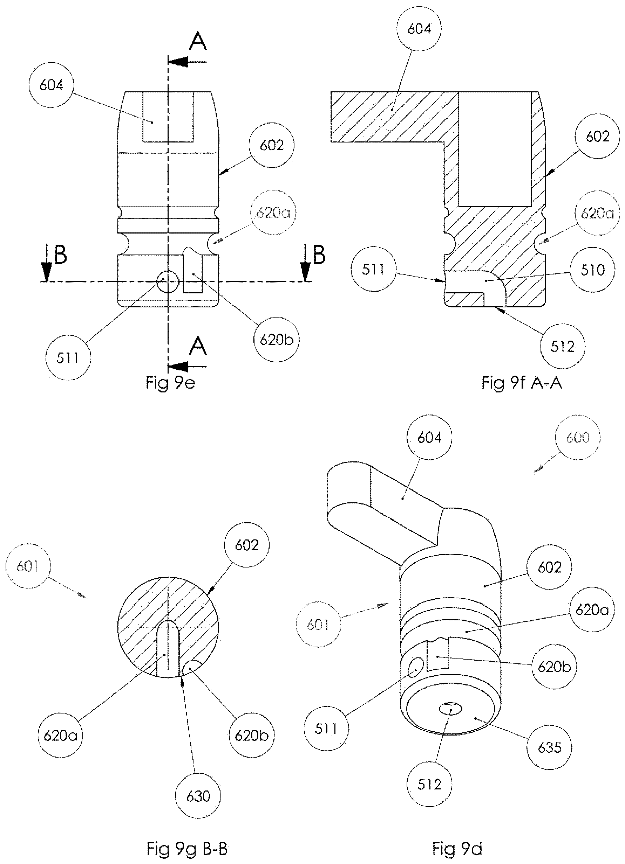

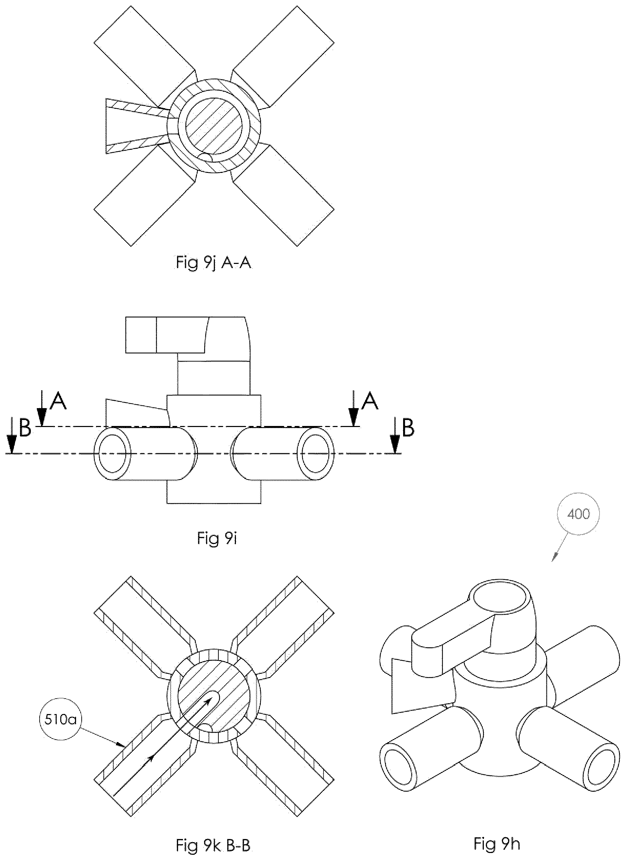

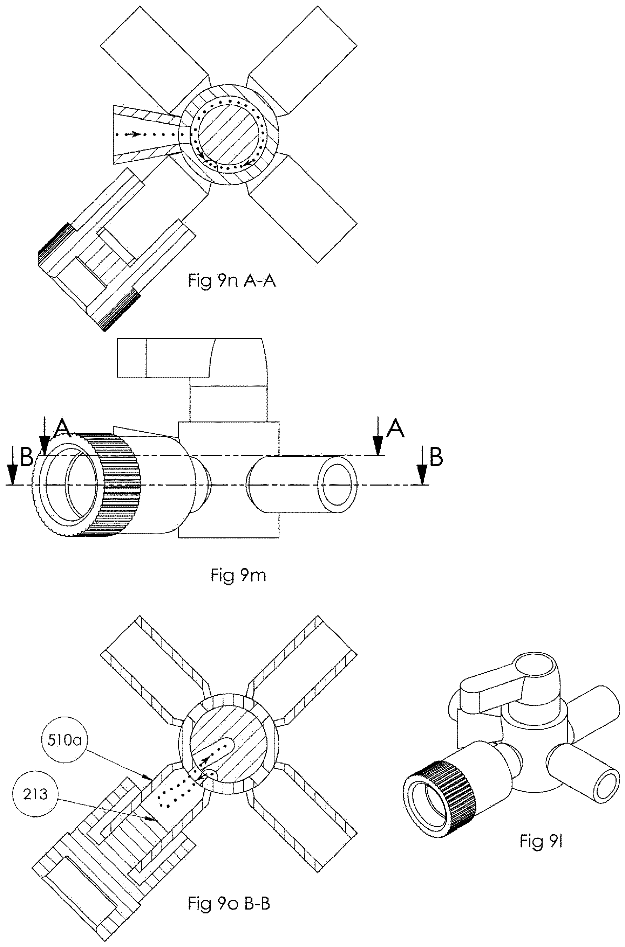

Fig. 9 illustrates a first embodiment of a multi-drug, multi-level valve 400 according to the inventive concept. Figs 9a-c illustrate a housing 500 of multi-drug valve 400, Figs 9d-g illustrate a valve member 600 of the multidrug valve and Figs 9h-k illustrate the assembled multi-drug valve 400.

The valve 400 comprises a cylindrical housing 500 (Figs. 9a-c) and a cylindrical valve member 600 (Figs. 9d-g). A cylindrical body 601 of the valve member is rotatably arranged in the housing 500. By cylindrical is here meant a cylinder-shape with constant radius. The housing 500 and the valve body 601 may have shapes other than cylindrical. For example, they may be frustoconical, comprised of several frustoconical parts, etc. More specifically, an outer cylindrical surface 602 of the valve body 601 engages sealingly with an inner cylindrical surface 504 of the housing 500, thereby creating an assembly which is airtight and prevents the flow of fluids. Preferably, the diameter of the outer cylindrical surface 602 of the valve member 600 is slightly larger than the inner cylindrical surface 504 of the housing 500 in order to create the sealed engagement mentioned above. The description above regarding other sealing methods and manufacturing aspects apply to the multi-drug embodiments also.

The housing 500 illustrated in Figs 9a-c comprises a cylindrical housing wall 502 which encloses an inner cavity 505 into which the body 601 of the valve member 600 is to be inserted. The cylindrical housing wall 502 comprises an outer envelope surface 606 and said inner cylindrical surface 604. The housing 600 comprises four primary inlets 510a-d and a secondary inlet 520. All drug inlets connects to the inner cavity 505 of the housing 500 via openings 515 in the housing wall 502 and may be integrally formed with the cylindrical housing wall 502 and shaped as a pipe or a stud. The outlet 520 also connects to the inner cavity of the housing 500 via an opening 525 in the housing wall 502 and may also be integrally formed with the cylindrical housing wall 502 and shaped as a pipe or a stud.

Since the primary inlets 510 are intended to normally be used for the administration of drugs and the secondary inlet 520 is intended to normally be used for the administration of a neutral fluid, such as a saline solution or saline fluid, these inlets will for explanation purposes be referred to as "the drug inlets 510a-d" and "the saline inlet 520", respectively without therefore limiting the scope of the claims to the use of any specific fluids.

In general, when implementing the inventive concept in a multi-drug valve, the valve will also be of multi-level design. The embodiment in Fig. 9 is a two-level design, the drug inlets 510a-d being arranged on a first common level and the saline inlet 520 being arranged on a different level.

In the embodiment in Fig. 9, the four drug inlets 510a-d are mutually spaced 90 degrees in the circumferential direction, and the saline inlet 520 is located between two of the inlets 510a and 510b.

Referring now to Figs 9d-g, the valve member 600 is integrally formed with said cylindrical body 601 and a handle 604 arranged outside the housing 500 by means of which the valve member 600 may be rotated by the user. The shape of the handle will in known manner allow the user to identify the position of the valve member 600.

The body 601 of the valve member 600 which is rotatably and sealingly arranged in the inner cavity 505 of housing 500 is provided with a primary channel 510. As will be discussed below, according to the inventive concept the primary channel 510 is intended for primarily for drugs and it will therefore for explanation purposes be referred to as "the drug channel 510" without therefore limiting the scope of the claims to the use of any specific fluids therein.

In this embodiment, the drug channel 510 is formed as a 90 degree bore or channel through the body 501 and presents first and second openings 511 and 512, respectively. In the illustrated embodiment, the drug channel 510 is a 90 degree channel configured to be fluidly connected to or aligned with a selected one of the inlets 510a-d in a drug position, by aligning the opening 611 of the drug channel 610 with an associated opening 515 in the housing wall 502. The drug channel 610 extends in an axial plane coinciding with the rotational axis C. The opening 612 of the drug channel 610 is centrally arranged in a bottom 635 of the valve member 600 and forms an outlet of the valve 400.

The body 601 of the valve member 600 further comprises a saline channel 620, which is divided into two parts: (i) a ring shaped recess 620a arranged in level with the inlet 520 which is open radially towards the inner cylindrical surface of the housing wall, and (ii) a recess or groove 620b extending axially from the annular saline recess 620a towards but not into the bottom 635 of the valve member 600. The axial saline groove 620b would typically be arranged on the same level as the opening 611 of the drug channel 610 and as the drug inlets 510a-d. A relatively small wall portion 630 integrally formed with the valve member 600 separates the axial saline groove 620b from the drug channel 510. In the assembled state, the radially outer edge of this wall portion 630 may seal against the inner cylindrical surface 504 of the housing 500, as in the previous embodiments.

The operation of the multi-drug valve 600 in Fig. 9 will now be described. Figs 9i-k illustrate one of four possible drug positions. In the illustrated position of the valve member 500, the opening 611 of the drug channel 610 is fully aligned with the opening 515 of the first drug inlet 510a. A drug fluid may then follow an essentially unrestricted flow path D from the first drug inlet 510a, into the aligned drug channel 510 and axially out from the valve 400 through the outlet opening 612 in the valve member 500. In this drug position, it will be seen that the wall portion 630 is located outside the opening 515 of the first inlet 510a and is in sealingly engagement with the housing wall, thus blocking any saline fluid that may be present in the saline groove 620b from flowing through the valve. The saline channel 620a-b is inactive in this position and only the drug channel 610 is used.

Figs 9I-o illustrate a priming/flushing position of the valve member 600. Compared to the drug position, the valve member 600 has now been rotated a relatively small angle clockwise to a position where the wall portion 630 bifurcates the opening 515 of the first drug inlet 510a. In this position, the first drug inlet 510a may be primed or flushed by a saline fluid, as shown by a dashed arrow F. In the priming/flushing position, the saline fluid will follow the following flow path:
Inlet 620 → housing wall opening 525 → ring-shaped saline channel 620a in valve member 600 → axial saline groove 620b in valve member 600 → housing wall opening 515 → drug inlet 510a → housing wall opening 515 → drug channel 610 and out through channel opening 612 at bottom 635 of valve member 600.

Similar to the previous embodiments, it will be noted that the drug flow D is less restricted by the valve than the priming/flushing flow F due to the full alignment of the drug channel opening 611 with the housing wall opening 515.. The embodiment in Fig. 9 does not have a dedicated saline position. In order to administer a saline fluid to the patient, the priming/flushing position may be used.

A typical operation sequence of the multi-drug valve 400 in Fig. 9 could be as follows (where an arrow → indicates a new valve position):
Priming → Drug #1 at 510a → Flushing 510a and then saline → Drug #2 at 510b → Flushing 510b and then saline → Drug #3 at 510c → Flushing 510c and then saline → Drug #4 → Flushing 510d and then saline.

It will be noted that this sequence may be obtained by rotating the valve member 600 in the same direction (clock-wise in the drawing) for each new step. The valve member does not have to be rotated in the reverse direction. Put in other words, a flushing position is available in all intermediate positions between two consecutive drug positions. It will also be noted that this configuration allows for a full unrestricted drug flow in all the different drug positions, since the drug channel 510 always may be fully aligned with one of the inlet openings 515.

### Second embodiment of multi-drug valve - Dedicated saline positions

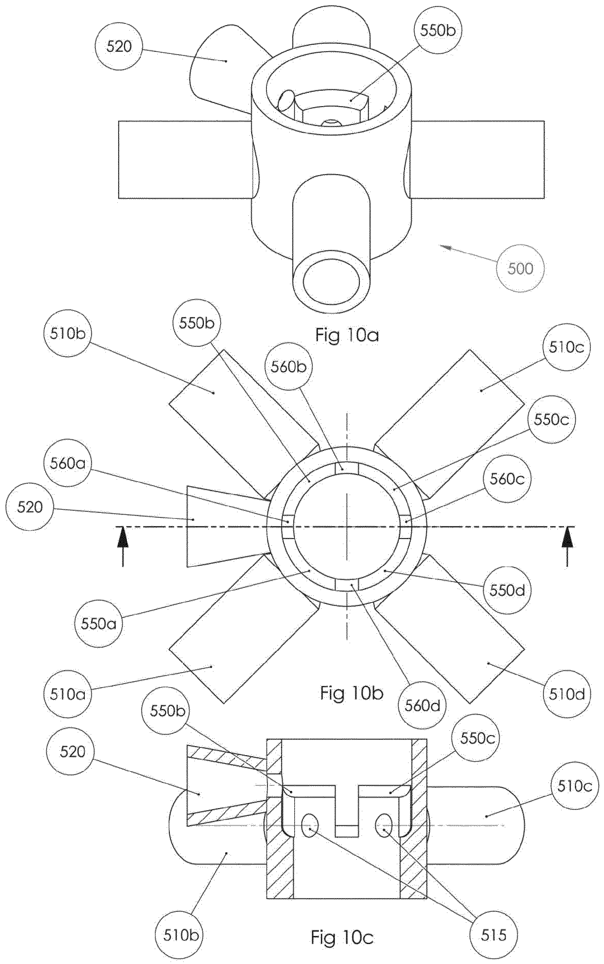

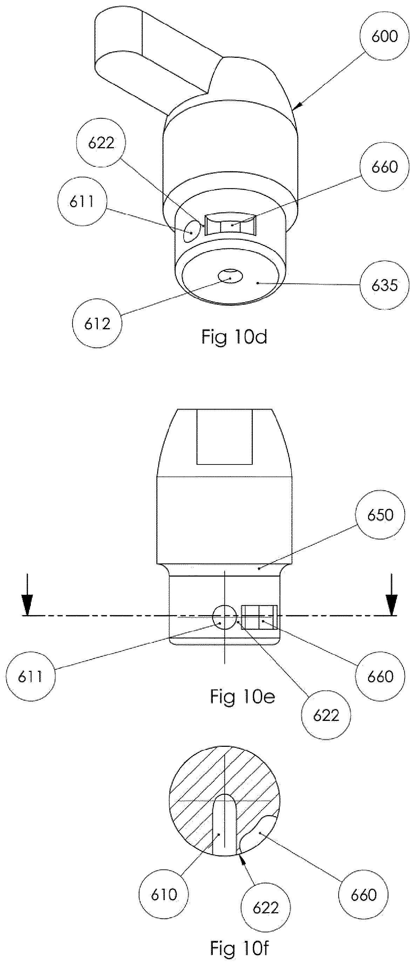

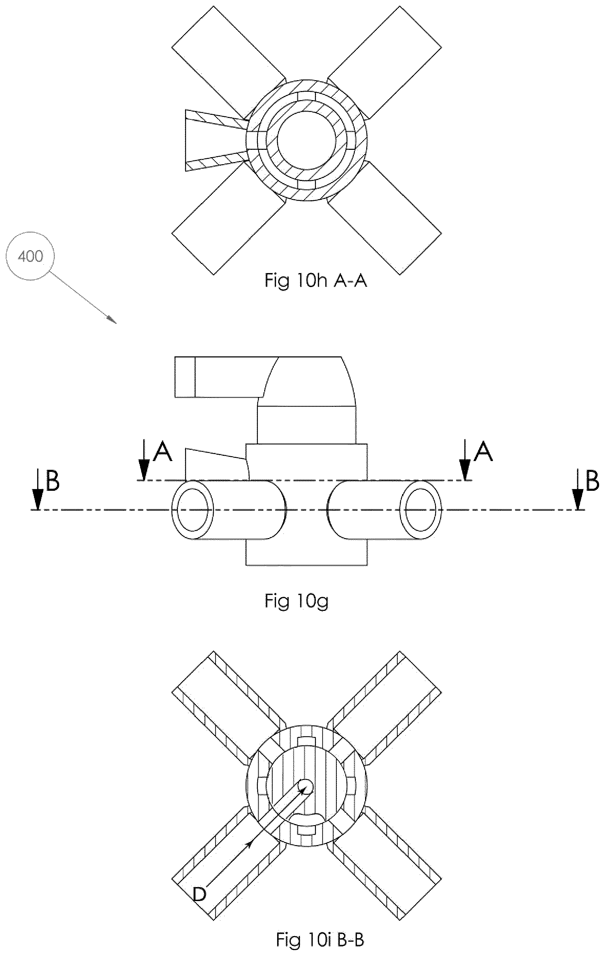

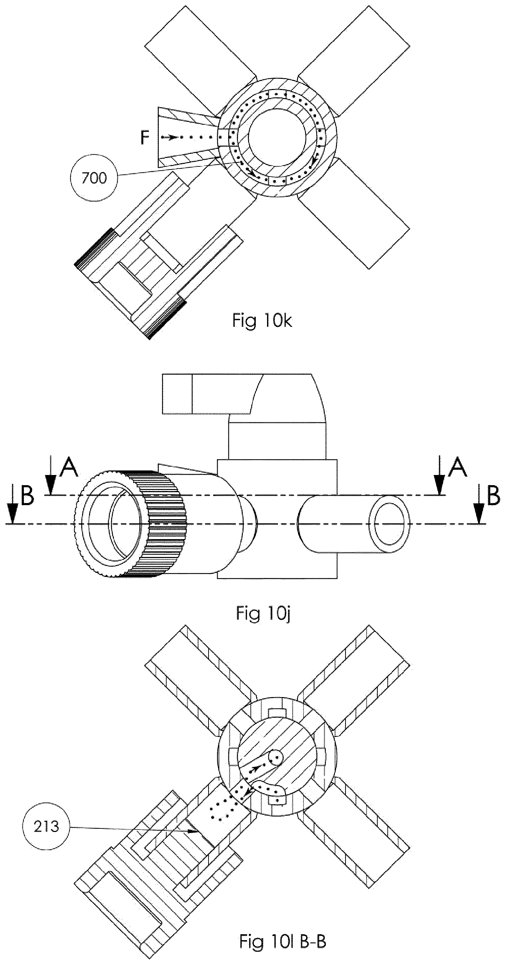

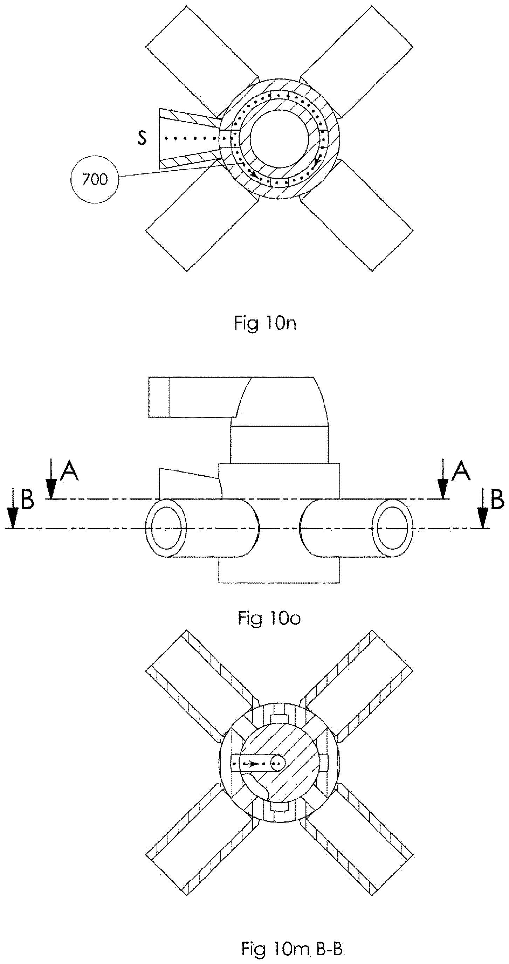

A second embodiment of a multi-drug valve according to the inventive aspect will now be described with reference to Fig. 10. This embodiment may be preferred over the embodiment in Fig. 9 in that the embodiment in Fig. 10 also presents dedicated intermediate saline positions in which the drug inlets are closed.

In the multi-drug valve 400 in Fig. 10, the housing 500 (Fig. 10a-c) is provided with an internal annular, downwardly sloping surface 550 facing towards the top or handle side of the valve and divided into four surface segments 550a-d. The surface segments 550a-d are separated by four openings or grooves 560a-d formed in the housing wall 502 and open radially towards to valve member 600. The grooves 560a-d extend in an axial direction from the surface segments 550a-d to a level corresponding to the level of the wall openings 515 at the drug inlets 510a-d.

The valve member 600 (Fig. 10d-f) comprises a drug channel 610 similar to the drug channel 610 in Fig. 9. Further, the valve member 600 comprises an annular, sloping surface 650 facing downwards towards the bottom 635 of the valve member 600. The annular surface 650 runs unbroken 360 degrees around the valve member 500. The valve member 600 also comprises an opening 660 in the body 601 which is located relatively close to the channel opening 611 and separated there from by a wall portion 622. The opening 660 opens radially towards the inner surface of the housing wall.

In the assembled state (Figs 10g-i), the valve member 600 will be so positioned in the housing 500 that the annular surface 650 of the valve member 600 and the four surface segments 550a-d of the housing 500 together form an annular channel 700 there between. The axial grooves 560a-d in the housing are open towards this annular channel 700. For the same reasons and conditions as above, the annular channel 700 is referred to as "the annular saline channel 700", the axial grooves 560 are referred to as the "axial saline grooves 560" and the opening 660 in the valve member 600 is referred to as "the saline opening 660"

The operation of the multi-drug valve 400 in Fig. 10 will now be described. Figs 10g-i illustrate one of four possible drug positions. The opening 611 of the drug channel 610 is fully aligned with the opening 515 of the first drug inlet 510a. A drug fluid may then follow an essentially unrestricted flow path D as follows:
First drug inlet 510a → opening 515 → drug channel 510 → axially out from the valve 400 trough the outlet opening 612

In the drug position, it will be noted that the wall portion 630 is located outside the opening 515 of the first inlet 510a and is in sealingly engagement with the housing wall, thus blocking any saline fluid that may be present in the saline opening 660 from flowing through the valve. Only the drug channel 610 is active in the drug position.

Figs 10j-k illustrate one of four priming/flushing positions of the valve member 600. Compared to the drug position, the valve member 600 has now been rotated a relatively small angle clockwise to a position where the wall portion 630 bifurcates the opening 515 of the first drug inlet 510a. In this position, the first drug inlet 510a may be primed or flushed by a saline fluid, as shown by a dashed arrow F. In the priming/flushing position, the saline fluid will follow the following flow path:
Inlet 620 → opening 525 → annular saline channel 700 → axial saline groove 560d → saline opening 660 → opening 515 → drug inlet 510a → opening 515 → drug channel 610 → out through channel opening 612 at bottom 635.

Similar to the previous embodiments, it will be noted that the drug flow D is less restricted than the priming/flushing flow F due to the full alignment of the drug channel opening 611 with the housing wall opening 515.

In contrast to the embodiment in Fig. 9, the embodiment in Fig. 10 has dedicated saline position for administration of a saline fluid. Figs 10m-o illustrate a saline position in which the valve member 600 has been rotated 90 degrees clockwise from the drug position. The opening 611 of the drug channel 610 is now aligned with the next axial saline groove 660a and a saline fluid may follow the following flow path S:
Inlet 620 → opening 525 → annular saline channel 700 → axial saline groove 560a → opening 515 → drug channel 610 → out through channel opening 612 at bottom 635.

It will be noted that all of the drug inlets 510a-d are closed in the saline position. That applies especially also to the drug inlet 510a which is open towards the opening 660 in valve member 600. Since opening 660 is in a position where it do not communicate with any of the axial saline grooves 560, the drug inlet 510a will be closed.

### Third multi-level embodiment - Enhance angular shift

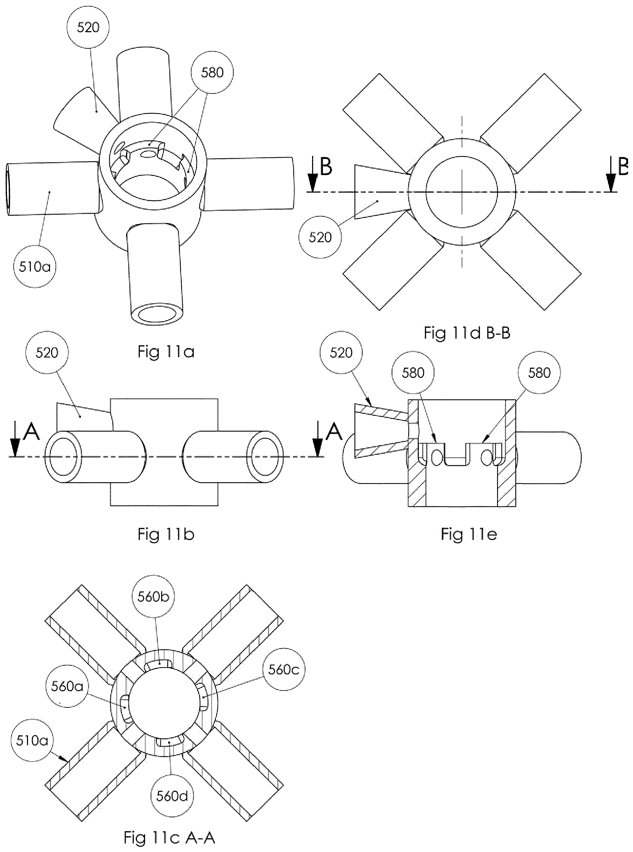

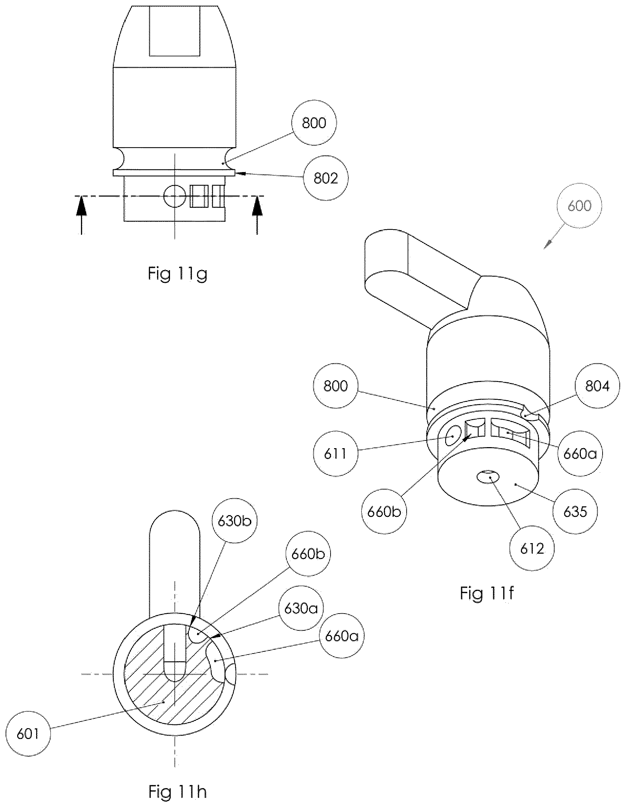

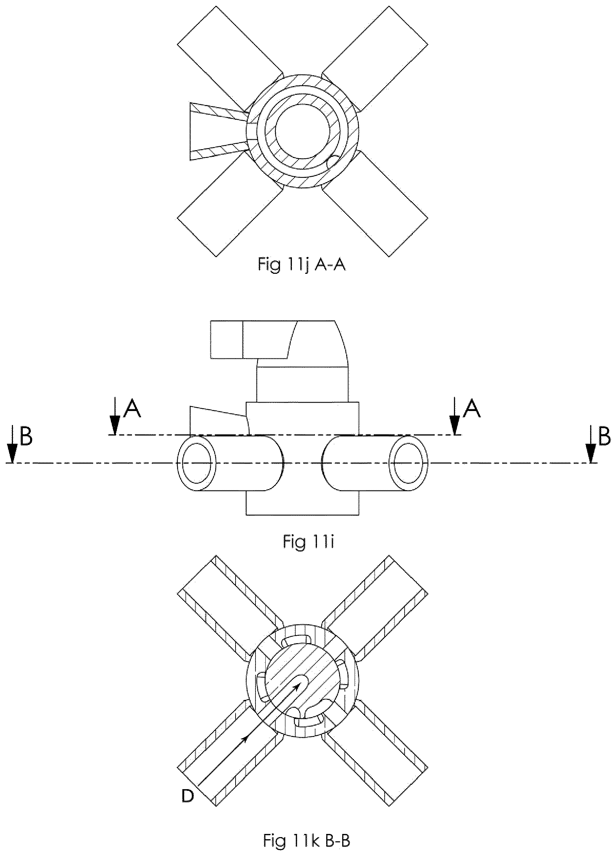

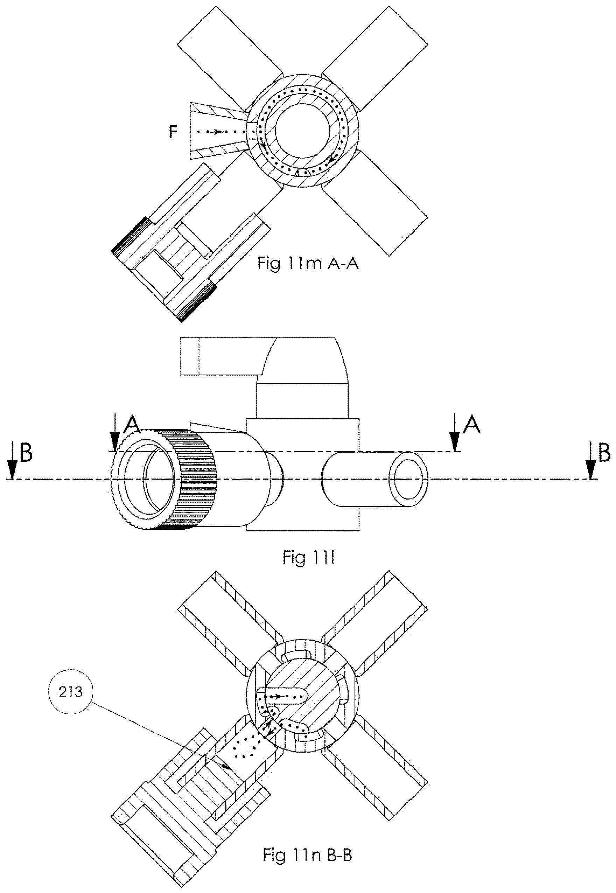

A third embodiment of a multi-level valve 400 according to the inventive concept will now be described with reference to Fig. 11. The embodiment according to Fig. 11 may be advantageous over the embodiments in Figs. 9 and 10 in that there is also an increased angular shift between the drug positions and the associated priming/flushing positions. Thereby, this embodiments avoids the potential drawback from a user perspective of having a relatively small angular shift between drug positions and priming/flushing positions.

As in the embodiment in Fig. 10, the housing 500 (Figs 11a-e) also comprises four axial saline grooves 560a-d but with the difference that these axial saline grooves 560a-d now are slightly angularly shifted so that they are not in a 45 degree shift relative the drug inlets 510a-d. Seen from the top of the housing 500, the grooves 560a-d are separated by four circumferentially extending surface segments 580 arranged in a common plane perpendicular to the rotational axis A.

As to the valve member 600 (Figs 11f-h), there are now two saline openings 660a and 660b, respectively, arranged in the body 601 in a common plane and radially open towards the outer cylindrical surface of the valve member 600. A relatively narrow first wall portion 630a separates the first saline opening 660a from the second saline opening 660b, and relatively narrow second wall portion 630b separates the second saline opening 660b from the drug channel 610. The radially outer edge of each wall portion 630a and 630b may be positioned to be in sealingly engagement with the inner surface of the housing 500.

Furthermore, the body 601 of the valve member 600 is provided with a radially open annular channel or recess 800, referred to as "the annular saline channel 800", an annular rim 802 which is in sealingly engagement with the inner housing wall, a peripheral recess 804 in the annular rim 802. In the assembled state, the annular rim 802 is resting on and is in sealingly engagement with the surface segments 580. As a result, any saline fluid present in the annular saline channel 800 will be blocked from flowing further down into the valve unless the peripheral recess 804 in the rim 802 is aligned with one of the axial saline grooves 560a-d in the housing.

In the drug position (Figs 11i-k), the situation is essentially the same as for the drug position in Fig. 10. Full alignment between the first drug inlet 510a and the drug channel 610 and there is a drug flow D from drug inlet 510a to valve outlet 612. The peripheral recess 804 is not aligned with any of the axial saline groove 560a-d and, therefore, the saline inlet 520 is essentially blocked. Only the drug channel 510 active in the drug position.

Now, as an important feature for this embodiment, compared to the previous minor angle shift there is now a substantial 45 degree rotational shift from the drug position (Figs 11i-k) to the priming/flushing position (Figs 11l-n) thanks to the provision of the two saline openings 660a and 660b operating in series or combination with two of the axial saline grooves 560a-d. In the illustrated priming-flushing position, the first wall portion 630a is located at the first drug inlet 510a and bifurcate the associated wall opening 515. The second wall portion 630b is in a position facing the next axial saline groove 560a. Thereby, none of the two wall portions 630a and 630b seal against the housing wall. Furthermore, the peripheral recess 804 in the rim 802 of the valve member 600 is now aligned with the axial saline groove 560d, allowing the saline fluid to enter down into the valve. The flow path F for the saline fluid in the priming/flushing position will be as follows:
Inlet 620 → opening 525 → annular saline channel 800 → peripheral recess 804 → axial saline groove 560d → first saline opening 660a → housing wall opening 515 → first drug inlet 510a → pass edge of first wall portion 630a → opening 515 → second saline opening 660b → axial saline groove 560a → pass edge of second wall portion 630b → drug channel 610 → out through channel opening or outlet 612 at bottom 635.

It will be noted that in the priming/flushing position, the flow path F goes via two of the axial saline grooves, one (560d) guiding the fluid in an axial direction and the other (560a) guiding the fluid in a circumferential direction. In the next sequential flushing position, groove 560a will instead guide the saline solution axially and the next groove 560b will guide the saline solution circumferentially.

It will be appreciated that the design in Fig. 11 using two saline openings in sequence in order to increase the angular shift between a drug position and an associated priming/flushing position may be applied also to valve designs having only one drug inlet and one saline inlet. This may be implemented in a multi-level design.

### Further design of multi-level valves

Having now illustrated and described various embodiments multi-level valves for multi-drug use, it will be appreciated that the teachings regarding the valve housing, the valve member and the channels/openings/grooves therein, and the operation of the valve, may be implemented also in the multi-level valves illustrated in Figs 3 to 6.

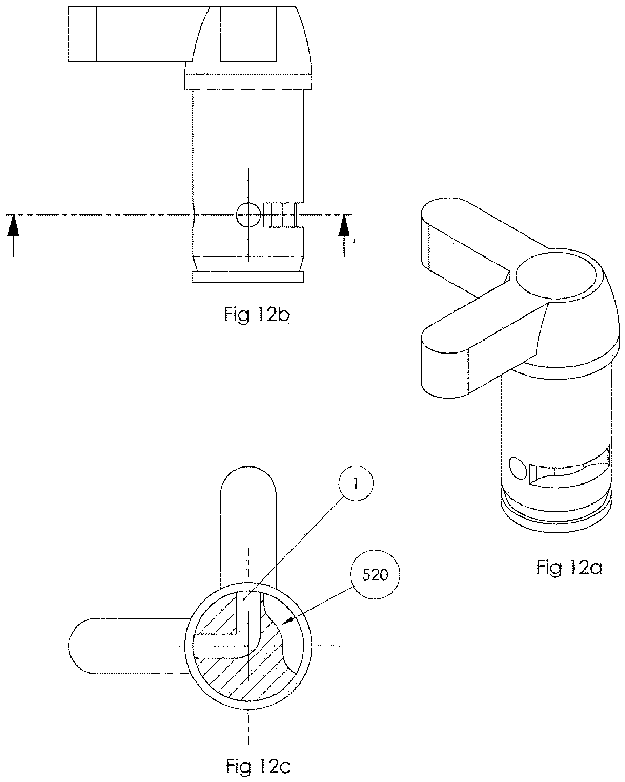

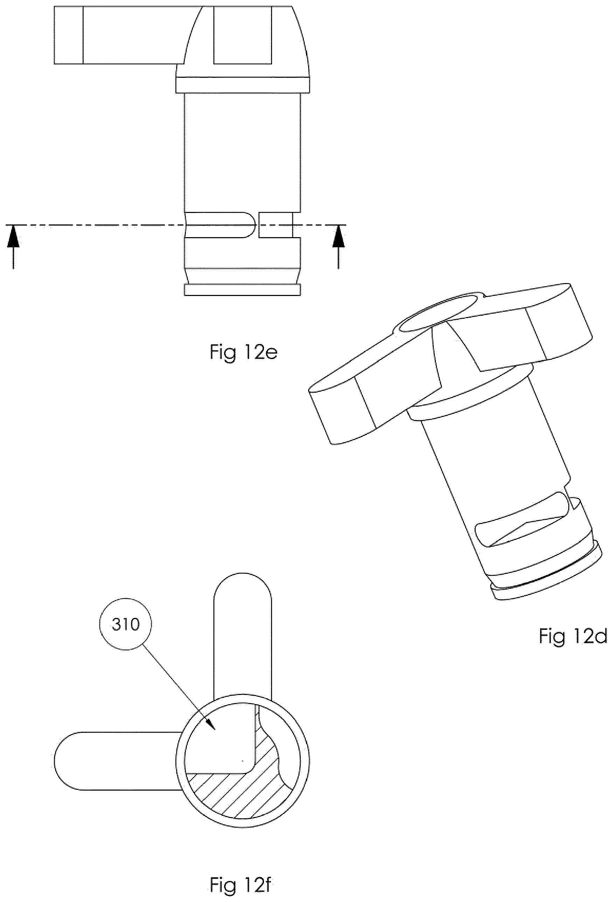

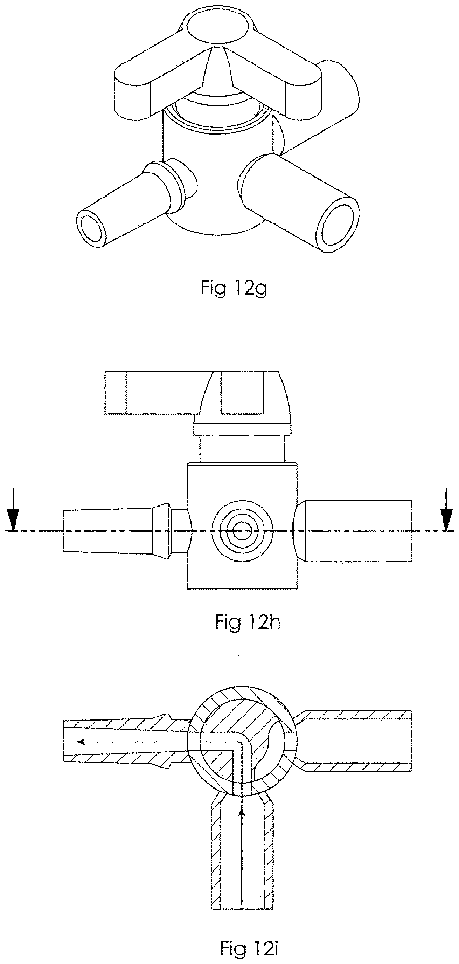

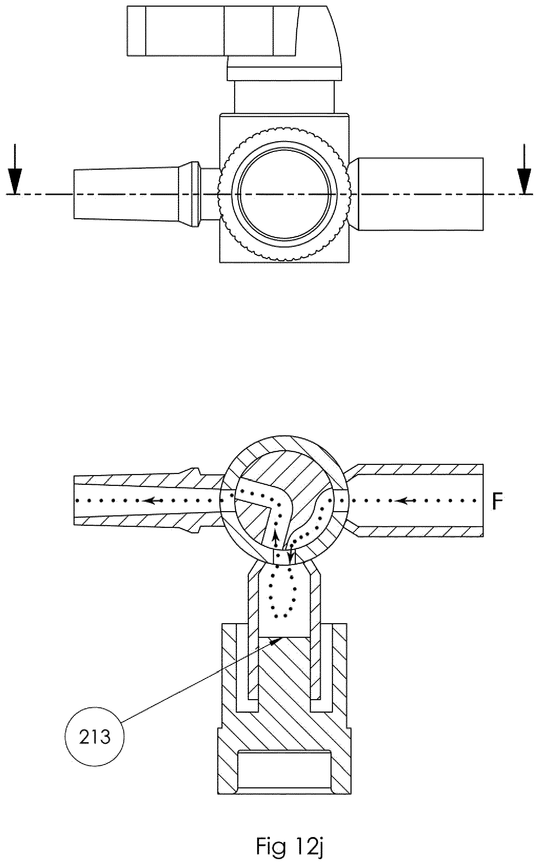

Fig. 12 illustrates a single-level embodiment of a valve according to the invention. Same reference numerals as above are used. This embodiment differs from the embodiment in Fig. 1 in that the saline channel 520 is shorter in the circumferential direction (about 90 degrees) and there is therefore no dedicated saline position. Fig. 12 also illustrates an alternative configuration of the valve member 300 in which the drug channel 310 is formed as an open recess like the saline channel 320, rather than a bore.

## Claims

1. A valve (100) for administration of drug fluids, such as cytostatics, comprising:
a valve housing (200) having at least one primary inlet (210), termed "drug inlet" for receiving a drug fluid and a secondary inlet (220), termed "saline inlet" for receiving a secondary fluid, such as a neutral fluid;
an outlet (230); and
a valve member (300) arranged to be positioned, e.g. rotated, into different valve positions in the housing (200) and having a primary channel (310) and a secondary channel (320),
wherein said different valve positions comprises:
- at least one primary valve position, termed "drug position", in which the drug inlet (210) is connected to the outlet (230) but not to the saline inlet (220), and
- at least one priming/flushing position, in which the saline inlet (220) is connected to the outlet (230) via the drug inlet (210) for priming or flushing the valve with said neutral fluid, and
wherein
in the drug position, the drug inlet (210) is connected to the outlet (230) via the primary channel (310) and there is no connection between the drug inlet (210) and secondary channel (320), and **characterized in that**
in the priming/flushing position, the saline inlet (220) is connected to the drug inlet (210) via the secondary channel (320) and the drug inlet (210) is connected to the outlet (230) via the primary channel (310).

2. A valve as claimed in any of the preceding claims, wherein the primary channel (310) is aligned with the drug inlet (210) and with the outlet (230) to a higher degree in the drug position than in the priming/flushing position, whereby a less restricted flow path is established from the drug inlet (210) to the outlet (230) in the drug position than in the priming/flushing position.

3. A valve as claimed in any of the preceding claims, wherein said different valve positions further comprises:
- at least one secondary valve position, termed "saline position", which differs from the drug position and the priming/flushing position and in which the saline inlet (220) is connected to the outlet (230) and there is no connection between the drug inlet (210) and the outlet (230).

4. A valve as claimed in claim 3, wherein, in the saline position, the saline inlet (220) is connected to the outlet (230) via the primary channel (310) and not connected to the secondary channel (320).

5. A valve as claimed in claim 3, wherein, in the saline position, the saline inlet (220) is connected to the outlet (230) via the secondary channel (320) and not connected to the primary channel (310).

6. A valve as claimed in any of the preceding claims,
wherein the valve member (300) further comprises a first wall portion (330; 622) arranged between the primary channel (310) and the secondary channel (320), and
wherein:
- in the priming/flushing position, said first wall portion (330) bifurcates the drug inlet (210),
- in the drug position, said first wall portion (330; 622) is in sealingly engagement with the housing (200) to prevent any fluid communication between the primary channel (310) and the secondary channel (320) past the first wall portion (330).

7. A valve (400) as claimed in any of claims 1 to 5,
wherein the valve member (600) further comprises:
- a fluid transfer channel (660b) arranged between the secondary channel (660a) and the primary channel (610),
- a first wall portion (630a) being arranged between the fluid transfer channel (660b) and the secondary channel (660a), and
- a second wall portion (630b) being arranged between the fluid transfer channel (660b) and the primary channel (610),
wherein the housing (500) further comprises an opening, termed by-pass opening (560a-d), which is formed in an inner surface of the housing (500) and opens towards the valve member (600), and
wherein
- in the drug position, at least the second wall portion (630b) is sealingly engaging the housing (500) to prevent any fluid communication between the primary channel (610) and the secondary channel (660a), and
- in the priming/flushing position, said first wall portion (630a) bifurcates the drug inlet (610), said the second wall portion (630b) bifurcates the by-pass opening and the primary channel (610) is connected to the by-pass opening, such that the neutral fluid in the priming/flushing position may flow in the following sequence: the secondary channel (660a) → the drug inlet (210) past the first wall portion (630a) → the fluid transfer channel (660b) → the by-pass opening past the second wall portion (630b) → the primary channel (610) → the outlet.

8. A valve (400) as claimed in any of the preceding claims,
wherein said at least one primary inlet comprises a plurality of primary inlets (510a-d), termed "drug inlets" for receiving a respective one of said drug fluids,
wherein said at least one primary valve position comprises:
- a plurality of primary valve positions, termed "drug positions" in each of which an associated one of the drug inlets (510a-d) is connected to the outlet via the drug channel (610), and
wherein said at least one priming/flushing position comprises:
- a plurality of priming/flushing positions, in which the saline inlet (520) is connected to the outlet via an associated one of the drug inlets (510a-d).

9. A valve as claimed in claim 8,
wherein said different valve positions comprises:
- a plurality of secondary positions, termed "saline positions", which differ from said plurality of drug positions and said plurality of priming/flushing positions and in each of which the saline inlet (520) is connected to the outlet and there is no connection between an associated drug inlet (510a-d) and the outlet.

10. A valve as claimed in claim 8 or 9, wherein the different valve positions are so arranged that there is one priming/flushing position between two drug positions and, if saline positions are available according to claim 9, there is also one saline position between two drug positions.

11. A valve as claimed in any of the preceding claims, wherein said inlets and the outlet are arranged in at least two different levels.

12. A valve as claimed in any of the preceding claims, wherein the outlet is arranged in a bottom of the valve and centered in relation to a rotational axis of the valve member.

13. A valve as claimed in any of the preceding claims, wherein said different valve positions are identifiable by a tactile response to a user.

## Patentansprüche

1. Ventil (100) zur Verabreichung von Arzneimittelfluiden, wie beispielsweise Zytostatika, umfassend:
ein Ventilgehäuse (200), das mindestens einen primären Einlass (210), der als "Arzneimitteleinlass" zum Empfangen eines Arzneimittelfluids bezeichnet wird, und einen sekundären Einlass (220) aufweist, der als "Kochsalzlösungseinlass" zur Aufnahme eines sekundären Fluids, wie beispielsweise eines neutralen Fluids, bezeichnet wird;
einen Auslass (230); und
ein Ventilelement (300), das eingerichtet ist, um in verschiedene Ventilpositionen in dem Gehäuse (200) positioniert, z. B. gedreht, zu werden und einen primären Kanal (310) und einen sekundären Kanal (320) aufweist, wobei die verschiedenen Ventilpositionen umfassen:
- mindestens eine primäre Ventilposition, die als "Arzneimittel-Position" bezeichnet wird, bei welcher der Arzneimitteleinlass (210) mit dem Auslass (230), jedoch nicht mit dem Kochsalzlösungseinlass (220) verbunden ist, und
- mindestens eine Füllen-/Spülen-Position, in welcher der Kochsalzlösungseinlass (220) über den Arzneimitteleinlass (210) mit dem Auslass (230) verbunden ist, um das Ventil mit dem neutralen Fluid zu füllen oder zu spülen, und
wobei
in der Arzneimittel-Position der Arzneimitteleinlass (210) über den primären Kanal (310) mit dem Auslass (230) verbunden ist und keine Verbindung zwischen dem Arzneimitteleinlass (210) und dem sekundären Kanal (320) vorhanden ist, **dadurch gekennzeichnet, dass**
in der Füllen-/Spülen-Position der Kochsalzlösungseinlass (220) über den sekundären Kanal (320) mit dem Arzneimitteleinlass (210) verbunden ist und der Arzneimitteleinlass (210) über den primären Kanal (310) mit dem Auslass (230) verbunden ist.

2. Ventil nach einem der vorhergehenden Ansprüche, wobei der primäre Kanal (310) mit dem Arzneimitteleinlass (210) und mit dem Auslass (230) in der Arzneimittelposition in einem höheren Maße ausgerichtet ist als in der Füllen-/Spülen-Position, wodurch in der Arzneimittelposition ein weniger eingeschränkter Strömungspfad von dem Arzneimitteleinlass (210) zu dem Auslass (230) als in der Füllen-/Spülen-Position eingerichtet wird.

3. Ventil nach einem der vorhergehenden Ansprüche, wobei die verschiedenen Ventilpositionen ferner umfassen:
- mindestens eine sekundäre Ventilposition, die als "Kochsalzlösungs-Position" bezeichnet wird, die sich von der Arzneimittelposition und der Füllen-/Spülen-Position unterscheidet und in welcher der Kochsalzlösungseinlass (220) mit dem Auslass (230) verbunden ist und keine Verbindung zwischen dem Arzneimitteleinlass (210) und dem Auslass (230) vorhanden ist.

4. Ventil nach Anspruch 3, wobei in der Kochsalzlösungsposition der Kochsalzlösungseinlass (220) über den primären Kanal (310) mit dem Auslass (230) verbunden ist und nicht mit dem sekundären Kanal (320) verbunden ist.

5. Ventil nach Anspruch 3, wobei in der Kochsalzlösungsposition der Kochsalzlösungseinlass (220) über den sekundären Kanal (310) mit dem Auslass (230) verbunden ist und nicht mit dem primären Kanal (320) verbunden ist.

6. Ventil nach einem der vorhergehenden Ansprüche,
wobei das Ventilelement (300) ferner einen ersten Wandabschnitt (330; 622) umfasst, der zwischen dem primären Kanal (310) und dem sekundären Kanal (320) angeordnet ist, und
wobei:
- in der Füllen-/Spülen-Position, der erste Wandabschnitt (330) den Arzneimitteleinlass (210) gabelförmig teilt,
- in der Arzneimittelposition der erste Wandabschnitt (330; 622) in abdichtendem Eingriff mit dem Gehäuse (200) steht, um jegliche Fluidverbindung zwischen dem primären Kanal (310) und dem sekundären Kanal (320) über den ersten Wandabschnitt (330) hinaus zu verhindern.

7. Ventil (400) nach einem der Ansprüche 1 bis 5, wobei das Ventilelement (600) ferner umfasst:
- einen Fluidübertragungskanal (660b), der zwischen dem sekundären Kanal (660a) und dem primären Kanal (610) angeordnet ist,
- einen ersten Wandabschnitt (630a), der zwischen dem Fluidübertragungskanal (660b) und dem sekundären Kanal (660a) angeordnet ist, und
- einen zweiten Wandabschnitt (630b), der zwischen dem Fluidübertragungskanal (660b) und dem primären Kanal (610) angeordnet ist,
wobei das Gehäuse (500) ferner eine Öffnung umfasst, die als Bypassöffnung (560a-d) bezeichnet wird, die in einer Innenfläche des Gehäuses (500) ausgebildet ist und sich in Richtung des Ventilelements (600) öffnet, und
wobei
- in der Arzneimittelposition, mindestens der zweite Wandabschnitt (630b) abdichtend mit dem Gehäuse (500) in Eingriff tritt, um jegliche Fluidverbindung zwischen dem primären Kanal (610) und dem sekundären Kanal (660a) zu verhindern, und
- in der Füllen-/Spülen-Position, der erste Wandabschnitt (630a) den Arzneimitteleinlass (610) gabelförmig teilt, der zweite Wandabschnitt (630b) die Bypassöffnung gabelförmig teilt und der primäre Kanal (610) mit der Bypassöffnung derart verbunden ist, dass das neutrale Fluid in der Füllen-/Spülen-Position in der folgenden Reihenfolge fließen kann: durch den sekundären Kanal (660a) → den Arzneimitteleinlass (210) bis hinter den ersten Wandabschnitt (630a) → den Fluidübertragungskanal (660b) → die Bypassöffnung bis hinter den zweiten Wandabschnitt (630b) → den primären Kanal (610) → zu dem Auslass.

8. Ventil (400) nach einem der vorhergehenden Ansprüche,
wobei der mindestens eine primäre Einlass eine Vielzahl von primären Einlässen (510a-d) umfasst, die als "Arzneimitteleinlässe" bezeichnet werden, um jeweils eines der Arzneimittelfluide zu empfangen,
wobei die mindestens eine primäre Ventilposition umfasst:
- eine Vielzahl von primären Ventilpositionen, die als "Arzneimittelpositionen" bezeichnet werden, in denen jeweils ein zugeordneter der Arzneimitteleinlässe (510a-d) über den Arzneimittelkanal (610) mit dem Auslass verbunden ist, und
wobei die mindestens eine Füllen-/Spülen-Position umfasst:
- eine Vielzahl von Füllen-/Spülen-Positionen, in denen der Kochsalzlösungseinlass (520) über einen zugeordneten der Arzneimitteleinlässe (510a-d) mit dem Auslass verbunden ist.

9. Ventil nach Anspruch 8,
wobei die verschiedenen Ventilpositionen umfassen:
- eine Vielzahl von sekundären Positionen, die als "Kochsalzlösungspositionen" bezeichnet werden, die sich von der Vielzahl von Arzneimittelpositionen und der Vielzahl von Füllen-/Spülen-Positionen unterscheiden und in denen jeweils der Kochsalzlösungseinlass (520) mit dem Auslass verbunden ist und keine Verbindung zwischen einem zugeordneten Kochsalzeinlass (510a-d) und dem Auslass vorhanden ist.

10. Ventil nach Anspruch 8 oder 9, wobei die verschiedenen Ventilpositionen so angeordnet sind, dass zwischen zwei Arzneimittelpositionen eine Füllen-/Spülen-Position vorhanden ist und, wenn Kochsalzlösungspositionen nach Anspruch 9 vorhanden sind, auch eine Kochsalzlösungsposition zwischen zwei Arzneimittelpositionen vorhanden ist.

11. Ventil nach einem der vorhergehenden Ansprüche, wobei die Einlässe und der Auslass in mindestens zwei verschiedenen Ebenen angeordnet sind.

12. Ventil nach einem der vorhergehenden Ansprüche, wobei der Auslass in einem Boden des Ventils angeordnet und in Bezug auf eine Drehachse des Ventilelements zentriert ist.

13. Ventil nach einem der vorhergehenden Ansprüche, wobei die verschiedenen Ventilpositionen durch eine taktile Rückmeldung für einen Benutzer identifizierbar sind.

## Revendications

1. Vanne (100) pour l'administration de fluides médicamenteux, tels que des cytostatiques, comprenant :
un boîtier de vanne (200) doté d'au moins une admission primaire (210), appelée « administration de médicament, destinée à recevoir un fluide médicamenteux et d'une admission secondaire (220), appelée « admission secondaire », destinée à recevoir un fluide secondaire, tel qu'un fluide neutre ;
une sortie (230) ; et
un élément vanne (300) conçu pour être positionné, c'est-à-dire tourné, dans différentes positions de vanne dans le boîtier (200) et doté d'un canal primaire (310) et d'un canal secondaire (320),
lesdites différentes positions de vanne comprenant :
- au moins une position de vanne primaire, appelé « position de médicament », dans laquelle l'admission de médicament (210) est connectée à la sortie (230) mais pas à l'admission de solution saline (220), et
- au moins une position d'apprêtage/de rinçage, dans laquelle l'admission de solution saline (220) est connectée à la sortie (230) via l'admission de médicament (210) pour apprêter ou rincer la vanne avec ledit fluide neutre, et
dans laquelle,
dans la position de médicament, l'admission de médicament (210) est connectée à la sortie (230) via le canal primaire (310) et il n'y a pas de connexion entre l'admission de médicament (210) et le canal secondaire (320), et **caractérisée en ce que**,
dans la position d'apprêtage/de rinçage, l'admission de solution saline (220) est connectée à l'admission de médicament (110) via le canal secondaire (320) et l'admission de médicament (210) est connectée à la sortie (230) via le canal primaire (310).

2. Vanne selon l'une quelconque des revendications précédentes, dans lequel le canal primaire (310) est aligné avec l'admission de médicament (210) et avec la sortie (230) à un degré plus élevé dans la position de médicament que dans la position d'apprêtage/de rinçage, ce qui a pour effet qu'il est établi dans la position de médicament un parcours d'écoulement moins restreint entre l'admission de médicament (210) et la sortie (230) que dans la position d'apprêtage/de rinçage.

3. Vanne selon l'une quelconque des revendications précédentes, dans laquelle lesdites différentes positions de vanne comprennent en outre :
- au moins une position de vanne secondaire, appelée « position de solution saline », qui diffère de la position de médicament et de la position d'apprêtage/de rinçage et dans laquelle l'admission de solution saline (220) est connectée à la sortie (230) et il n'y a pas de connexion entre l'admission de médicament (210) et la sortie (230).

4. Vanne selon la revendication 3, dans laquelle, dans la position de solution saline, l'admission de solution saline (220) est connectée à la sortie (230) via le canal primaire (310) et n'est pas connectée au canal secondaire (320).

5. Vanne selon la revendication 3, dans laquelle, dans la position de solution saline, l'admission de solution saline (220) est connectée à la sortie (230) via le canal secondaire (320) et n'est pas connectée au canal primaire (310).

6. Vanne selon l'une quelconque des revendications précédentes, dans laquelle l'élément vanne (300) comprend en outre une première section de paroi (330 ; 622) disposée entre le canal primaire (310) et le canal secondaire (320), et
- dans la position d'apprêtage/de rinçage, ladite section de paroi (330) fait bifurquer l'admission de médicament (210),
- dans la position de médicament, ladite première section de paroi (330 ; 622) est en engagement hermétique avec le boîtier (200) pour empêcher toute communication fluidique entre le canal primaire (310) et le canal secondaire (320) après la première section de paroi (330).

7. Vanne (400) selon l'une quelconque des revendications 1 à 5,
dans laquelle l'élément vanne (600) comprend en outre :
- un canal de transfert de fluide (660b) disposé entre le canal secondaire (660a) et le canal primaire (610),
- une première section de paroi (630a) étant disposée entre le canal de transfert de fluide (660b) et le canal secondaire (660a), et
- une seconde section de paroi (630b) étant disposée entre le canal de transfert de fluide (660b) et le canal primaire (610),
le boîtier (500) comprend en outre une ouverture, appelée ouverture de contournement (560a-d), qui est formée dans une surface intérieure du boîtier (500) s'ouvre vers l'élément vanne (600), et
- dans la position de médicament, au moins la seconde section de paroi (630b) est engagée hermétiquement dans le boîtier (500) pour empêcher toute communication fluidique entre le canal primaire (610) et le canal secondaire (660a), et
- dans la position d'apprêtage/de rinçage, ladite première section de paroi (630a) fait bifurquer l'admission de médicament (610), ladite seconde section de paroi (630b) fait bifurquer l'ouverture de contournement et le canal primaire (610) est connecté à l'ouverture de contournement, de sorte que le fluide neutre dans la position d'apprêtage/de rinçage peut couler dans la fréquence suivante : le canal secondaire (660a)→ l'admission de médicament (210) après la première section de paroi (630a)→ le canal de transfert de fluide (660b)→ l'ouverture de contournement après la seconde section de paroi (630b)→le canal primaire (610)→ la sortie.

8. Vanne (400) selon l'une quelconque des revendications précédentes, dans laquelle ladite au moins une admission primaire comprend une pluralité d'admissions primaires (510a-d), appelée « admission de médicament », pour recevoir l'un respectif parmi lesdits fluides médicamenteux,
ladite au moins une position de vanne primaire comprend :
- une pluralité de positions de vanne primaire, appelées « position de médicament », dans chacune desquelles une admission associée parmi les admissions de médicament (510a-d) est connectée à la sortie vient le canal à médicament (610), et
ladite au moins une position d'apprêtage/de rinçage comprend :
- une pluralité de positions d'apprêtage/de rinçage, dans lesquelles l'admission de solution saline (520) est connectée à la sortie via une admission associée parmi les admissions de médicament (510a-d).

9. Vanne selon la revendication 8,
dans laquelle lesdites différentes positions de vanne comprennent :
- une pluralité de positions secondaires, appelées « positions de solution saline » qui diffèrent de ladite pluralité de positions de médicament et de ladite pluralité de position d'apprêtage de rinçage et dans chacune desquelles l'admission de solution saline (520) est connectée à la sortie et il n'y a pas de connexion entre une admission de médicament associée (510a-d) et la sortie.

10. Vanne selon la revendication 8 ou 9, dans laquelle les différentes positions de vanne sont agencées de manière à ce qu'il y ait une position d'apprêtage/de rinçage entre deux positions de médicament et, si les positions de solution saline sont disponibles selon la revendication 9, il y a aussi une seule position de solution saline entre deux positions de médicament.

11. Vanne selon l'une quelconque des revendications précédentes, dans laquelle lesdites admissions et la sortie sont disposées à au moins deux niveaux différents.

12. Vanne selon l'une quelconque des revendications précédentes, dans laquelle la sortie est disposée dans un fond de la vanne et centrée par rapport à un axe de rotation de l'élément vanne.

13. Vanne selon l'une quelconque des revendications précédentes, dans laquelle lesdites positions de vanne différentes sont identifiables par une réponse tactile à un utilisateur.
